# EUROPEAN PATENT SPECIFICATION

(11) **EP 3 049 105 B1**
(45) Date of publication and mention of the grant of the patent: **30.12.2020**
(21) Application number: 14781775.3
(22) Date of filing: 19.09.2014
(51) Int. Cl.: A61K 39/02

(54) **VACCINE**
IMPFSTOFF
VACCIN

(30) Priority: 19.09.2013 US 201361879959 P
(43) Date of publication of application: 03.08.2016
(73) Proprietor: Moredun Research Institute, Bush Loan Penicuik Midlothian EH26 0PZ (GB)
(72) Inventor: FONTAINE, Michael, Christopher, Penicuik Midlothian EH26 0PZ (GB); SMITH, David, George, Emslie, Penicuik Midlothian EH26 0PZ (GB); FITZPATRICK, Julie, Lydia, Penicuik Midlothian EH26 0PZ (GB); DONACHIE, William, Penicuik Midlothian EH26 0PZ (GB); JAGLARZ, Anita, Dorata, Penicuik Midlothian EH26 0PZ (GB)
(74) Representative: Gibbs, Richard
(86) International application number: PCT/US2014/056591
(87) International publication number: WO 2015/042423

(56) References cited:
- EP-A1- 2 023 143
- WO-A1-2010/041056
- WARD PHILIP N ET AL: "Evidence for niche adaptation in the genome of the bovine pathogen Streptococcus uberis", BMC GENOMICS, BIOMED CENTRAL LTD, LONDON, UK, vol. 10, no. 1, 28 January 2009 (2009-01-28), page 54, XP021047972, ISSN: 1471-2164, DOI: 10.1186/1471-2164-10-54 & DATABASE EMBL [Online] 28 January 2009 (2009-01-28), Database accession no. AM946015 & DATABASE UniProt [Online] 28 July 2009 (2009-07-28), "RecName: Full=Elongation factor Tu {ECO:0000255|HAMAP-Rule:MF_00118}; Short=EF-Tu {ECO:0000255|HAMAP-Rule:MF_00118};", retrieved from EBI accession no. UNIPROT:B9DRL9 Database accession no. B9DRL9
- PRADO M E ET AL: "Vaccination of dairy cows with recombinant Streptococcus uberis adhesion molecule induces antibodies that reduce adherence to and internalization of S. uberis into bovine mammary epithelial cells.", VETERINARY IMMUNOLOGY AND IMMUNOPATHOLOGY 15 JUN 2011, vol. 141, no. 3-4, 15 June 2011 (2011-06-15), pages 201-208, XP002737876, ISSN: 1873-2534
- M DENIS ET AL: "Vaccines against bovine mastitis in the New Zealand context: What is the best way forward?", NEW ZEALAND VETERINARY JOURNAL, vol. 57, no. 3, 1 June 2009 (2009-06-01), pages 132-140, XP055179066, ISSN: 0048-0169, DOI: 10.1080/00480169.2009.36892

### SEQUENCE LISTING

<110> ZOETIS LLC Fontaine, Michael Christopher Smith, David George Emslie Fitzpatrick, Julie Lydia Donachie, William Jaglarz, Anita Dorota
<120> VACCINE
<130> ZP000074
<150> 61/879959
   <151> 2013-09-19
<160> 8
<170> PatentIn version 3.5
<210> 1
   <211> 944
   <212> DNA
   <213> ferrichrome binding protein
<400> 1 
atgggcagca gccatcatca tcatcatcac agcagcggcc tggtgccgcg cggcagccat 60

atgctcgaga tgtcacaaag cacaaagcaa gaagatcata aaacaaaact atcacaaatg 120

ccaaagatct ctggttttac ctataaaggg aaggtaccag aaaaccctaa aagagtagtt 180

agtttatctt caacctacac cggttatttg gcaaagctcg atatcccact agttggaatc 240

acttcttatg atcacaaaaa tcccgtctta aagaaataca tcaaggatgc taaagttgtc 300

tctgcaaccg acctagaaag cattacggcc ttggaacctg atttaattat tgtgggttca 360

aatgaagaaa atatcagtca attagctgaa atcgctcccc ttatttccat tgaataccgc 420

aaacatgact atttacaggt attctcagat tttggtaaag tctttaacaa aaccaaagaa 480

accgacaaat ggttacagga atggaaaaca aaaacagctt cttttgaaag tgacgttaaa 540

gcagttacag gtaataatgc tacctttacc ataatgggat tatatgagaa agatatctat 600

cttttcggta aagattgggg tcgtggtggt gaaatcattc accaagcctt ccaatatcaa 660

gctccagaaa aagtaaaaat ggaggttttc ccaaaaggct atttgtccat ttcacaagaa 720

gttcttccag attatattgg tgattatgtc gttgtcgctg cagaggatga aaaaacaggt 780

tcttctcttt atgaaagtga cctttggaaa aatataccag ccgttcaaaa aaatcatgtc 840

ataaatgtta atgcgaatac cttttatttc actgaccctc tgtcattaga gtatgaatta 900

aaaaccttaa cggatgctat cttgactcag aaaactcaca acta 944
<210> 2
   <211> 314
   <212> PRT
   <213> Artificial Sequence
<220>
   <223> rSUB0423 ferrichrome binding protein
<400> 2 
Met Gly Ser Ser His His His His His His Ser Ser Gly Leu Val Pro
1               5                   10                  15

Arg Gly Ser His Met Leu Glu Met Ser Gln Ser Thr Lys Gln Glu Asp
            20                  25                  30

His Lys Thr Lys Leu Ser Gln Met Pro Lys Ile Ser Gly Phe Thr Tyr
        35                  40                  45

Lys Gly Lys Val Pro Glu Asn Pro Lys Arg Val Val Ser Leu Ser Ser
    50                  55                  60

Thr Tyr Thr Gly Tyr Leu Ala Lys Leu Asp Ile Pro Leu Val Gly Ile
65                  70                  75                  80

Thr Ser Tyr Asp His Lys Asn Pro Val Leu Lys Lys Tyr Ile Lys Asp
                85                  90                  95

Ala Lys Val Val Ser Ala Thr Asp Leu Glu Ser Ile Thr Ala Leu Glu
            100                 105                 110

Pro Asp Leu Ile Ile Val Gly Ser Asn Glu Glu Asn Ile Ser Gln Leu
        115                 120                 125

Ala Glu Ile Ala Pro Leu Ile Ser Ile Glu Tyr Arg Lys His Asp Tyr
    130                 135                 140

Leu Gln Val Phe Ser Asp Phe Gly Lys Val Phe Asn Lys Thr Lys Glu
145                 150                 155                 160

Thr Asp Lys Trp Leu Gln Glu Trp Lys Thr Lys Thr Ala Ser Phe Glu
                165                 170                 175

Ser Asp Val Lys Ala Val Thr Gly Asn Asn Ala Thr Phe Thr Ile Met
            180                 185                 190

Gly Leu Tyr Glu Lys Asp Ile Tyr Leu Phe Gly Lys Asp Trp Gly Arg
        195                 200                 205

Gly Gly Glu Ile Ile His Gln Ala Phe Gln Tyr Gln Ala Pro Glu Lys
    210                 215                 220

Val Lys Met Glu Val Phe Pro Lys Gly Tyr Leu Ser Ile Ser Gln Glu
 
225                     230                     235                     240

Val Leu Pro Asp Tyr Ile Gly Asp Tyr Val Val Val Ala Ala Glu Asp
                245                     250                     255

Glu Lys Thr Gly Ser Ser Leu Tyr Glu Ser Asp Leu Trp Lys Asn Ile
            260                     265                     270

Pro Ala Val Gln Lys Asn His Val Ile Asn Val Asn Ala Asn Thr Phe
        275                     280                     285

Tyr Phe Thr Asp Pro Leu Ser Leu Glu Tyr Glu Leu Lys Thr Leu Thr
    290                     295                     300

Asp Ala Ile Leu Thr Gln Lys Thr His Asn
305                     310
<210> 3
   <211> 1265
   <212> DNA
   <213> Artificial Sequence
<220>
   <223> rSUB0604 elongation factor Tu
<400> 3 
tgggcagcag ccatcatcat catcatcaca gcagcggcct ggtgccgcgc ggcagccata 60

tgctcgagat ggcaaaagaa aaatacgatc gtagtaaacc ccacgttaac attggtacaa 120

ttggacacgt tgaccacggt aaaactactt tgacagctgc aattacaact gtacttgctc 180

gtcgcttacc aacttcagtt aaccaaccaa aagattacgc ttctatcgat gctgctccag 240

aagagcgcga acgcggaatc actatcaaca ctgcacacgt tgagtacgaa actgaaactc 300

gtcactatgc ccacattgat gccccaggac acgcggacta tgttaaaaac atgatcactg 360

gtgctgccca aatggacgga gctatccttg ttgttgcatc aactgatgga ccaatgccac 420

aaactcgtga gcacatcctt ctttcacgcc aagttggtgt taaacacctt atcgttttca 480

tgaacaaaat cgaccttgtt gacgatgaag aattgcttga attagttgaa atggaaatcc 540

gtgaccttct ttcagaatac gatttcccag gtgatgacct accagttatc caaggttcag 600

ctcttaaagc tcttgaaggt gattctaaat acgaagacat catcatggaa ttgatgaaaa 660

ctgctgatga gtatattcca gaaccagaac gtgatacaga caaaccatta cttcttccag 720

tcgaagacgt attctcaatc acaggtcgtg gtactgtagc ttcaggacgt atcgatcgtg 780

gtactgttcg tgtcaacgac gaaattgaaa tcgttggtat caaagaagaa actaaaaaag 840

cagttgttac tggtgttgaa atgttccgta aacaacttga cgaaggtctt gcaggagata 900

acgtaggtat ccttcttcgt ggtgttcaac gtgacgaaat cgaacgtgga caagttattg 960
 
ctaaaccagg ttcaatcaac ccacacacta aattcaaagg tgaagtttac atcctttcta 1020

aagatgaagg tggacgtcat actccattct tcaacaacta ccgtcctcaa ttctatttcc 1080

gtacaactga cgtaacaggt tcaatcgaac ttccagctgg tactgaaatg gtaatgcctg 1140

gtgataacgt gacaatcagc gttgagttga tccacccaat cgccgttgaa caaggtacta 1200

ctttctcaat ccgtgaaggt ggacgtactg ttggttcagg tattgtttca gaaatcgaag 1260

cttaa 1265
<210> 4
   <211> 421
   <212> PRT
   <213> Artificial Sequence
<220>
   <223> rSUB0604 elongation factor Tu
<400> 4 
Met Gly Ser Ser His His His His His His Ser Ser Gly Leu Val Pro
1               5                   10                  15

Arg Gly Ser His Met Leu Glu Met Ala Lys Glu Lys Tyr Asp Arg Ser
            20                  25                  30

Lys Pro His Val Asn Ile Gly Thr Ile Gly His Val Asp His Gly Lys
        35                  40                  45

Thr Thr Leu Thr Ala Ala Ile Thr Thr Val Leu Ala Arg Arg Leu Pro
    50                  55                  60

Thr Ser Val Asn Gln Pro Lys Asp Tyr Ala Ser Ile Asp Ala Ala Pro
65                  70                  75                  80

Glu Glu Arg Glu Arg Gly Ile Thr Ile Asn Thr Ala His Val Glu Tyr
                85                  90                  95

Glu Thr Glu Thr Arg His Tyr Ala His Ile Asp Ala Pro Gly His Ala
            100                 105                 110

Asp Tyr Val Lys Asn Met Ile Thr Gly Ala Ala Gln Met Asp Gly Ala
        115                 120                 125

Ile Leu Val Val Ala Ser Thr Asp Gly Pro Met Pro Gln Thr Arg Glu
    130                 135                 140

His Ile Leu Leu Ser Arg Gln Val Gly Val Lys His Leu Ile Val Phe
145                 150                 155                 160
 
Met Asn Lys Ile Asp Leu Val Asp Asp Glu Glu Leu Leu Glu Leu Val
                165                 170                 175

Glu Met Glu Ile Arg Asp Leu Leu Ser Glu Tyr Asp Phe Pro Gly Asp
            180                 185                 190

Asp Leu Pro Val Ile Gln Gly Ser Ala Leu Lys Ala Leu Glu Gly Asp
        195                 200                 205

Ser Lys Tyr Glu Asp Ile Ile Met Glu Leu Met Lys Thr Ala Asp Glu
    210                 215                 220

Tyr Ile Pro Glu Pro Glu Arg Asp Thr Asp Lys Pro Leu Leu Leu Pro
225                 230                 235                 240

Val Glu Asp Val Phe Ser Ile Thr Gly Arg Gly Thr Val Ala Ser Gly
                245                 250                 255

Arg Ile Asp Arg Gly Thr Val Arg Val Asn Asp Glu Ile Glu Ile Val
            260                 265                 270

Gly Ile Lys Glu Glu Thr Lys Lys Ala Val Val Thr Gly Val Glu Met
        275                 280                 285

Phe Arg Lys Gln Leu Asp Glu Gly Leu Ala Gly Asp Asn Val Gly Ile
    290                 295                 300

Leu Leu Arg Gly Val Gln Arg Asp Glu Ile Glu Arg Gly Gln Val Ile
305                 310                 315                 320

Ala Lys Pro Gly Ser Ile Asn Pro His Thr Lys Phe Lys Gly Glu Val
                325                 330                 335

Tyr Ile Leu Ser Lys Asp Glu Gly Gly Arg His Thr Pro Phe Phe Asn
            340                 345                 350

Asn Tyr Arg Pro Gln Phe Tyr Phe Arg Thr Thr Asp Val Thr Gly Ser
        355                 360                 365

Ile Glu Leu Pro Ala Gly Thr Glu Met Val Met Pro Gly Asp Asn Val
    370                 375                 380

Thr Ile Ser Val Glu Leu Ile His Pro Ile Ala Val Glu Gln Gly Thr
385                 390                 395                 400

Thr Phe Ser Ile Arg Glu Gly Gly Arg Thr Val Gly Ser Gly Ile Val
                405                 410                 415
 
Ser Glu Ile Glu Ala
            420
<210> 5
   <211> 1041
   <212> DNA
   <213> Artificial Sequence
<220>
   <223> rSUB0950 lipoprotein
<400> 5 
atgggcagca gccatcatca tcatcatcac agcagcggcc tggtgccgcg cggcagccat 60

atgctcgaga tggatagcaa agatgctaaa acagatttaa aagctgctat tgttactgat 120

acaggtggtg ttgatgataa atcatttaac caatctgctt gggaaggttt agaagcttgg 180

ggtaaagaaa atggcttaaa aaaaggtgct ggtttcgact acttccaatc aaatagtgaa 240

tcagaatatg ctactaatct tgacactgct gtctcaagtg gttataacgt agtatatgga 300

atcggatttg cccttaaaga tgcaattgat aaagctgctg gtgacaatag tgatgttaac 360

tatattatcg ttgacgatgt catcgaagga aaagataatg ttgcaagtgt aacttttgcg 420

gataacgaag ctgcttatct tgctggtatt gctgcagcta aaactacaaa aactaaagta 480

gtaggttttg taggtggtat ggaaggtact gttatcactc gttttgaaaa aggttttgag 540

gcgggagtga aatcagttga tgattctatc caaatcaaag ttgactacgc tggatcattt 600

ggtgatgctg ctaaaggtaa aacaattgcc gcagctcaat atgcaggtgg tgctgacgtt 660

atttatcaag ccgctggtgg tactggagca ggtgtcttca atgaagctaa agctgtaaat 720

gagaaaaaag atgaagctga taaagtttgg gtaatcggtg tagaccgtga ccaaaaagag 780

gaaggtaaat acacttcaaa agacggtaaa gaatctaact ttgttctagc atcttcaatt 840

aaacaagttg gtaaatctgt acaactgatt aacaaacttg ttactgataa aaaattccct 900

ggtggaaaaa caactgttta tggattaaaa gatggtggtg ttgatattgc aacaacaaac 960

ctttctgatg atgctataaa agctgttaaa gaagctaaag aaaaaattat ttctggcgat 1020

gtaaaagttc ctgaaaaata a 1041
<210> 6
   <211> 346
   <212> PRT
   <213> Artificial Sequence
<220>
   <223> rSUB0950 lipoprotein
<400> 6 Met Gly Ser Ser His His His His His His Ser Ser Gly Leu Val Pro 
1               5                   10                  15

Arg Gly Ser His Met Leu Glu Met Asp Ser Lys Asp Ala Lys Thr Asp
            20                  25                  30

Leu Lys Ala Ala Ile Val Thr Asp Thr Gly Gly Val Asp Asp Lys Ser
        35                  40                  45

Phe Asn Gln Ser Ala Trp Glu Gly Leu Glu Ala Trp Gly Lys Glu Asn
    50                  55                  60

Gly Leu Lys Lys Gly Ala Gly Phe Asp Tyr Phe Gln Ser Asn Ser Glu
65                  70                  75                  80

Ser Glu Tyr Ala Thr Asn Leu Asp Thr Ala Val Ser Ser Gly Tyr Asn
                85                  90                  95

Val Val Tyr Gly Ile Gly Phe Ala Leu Lys Asp Ala Ile Asp Lys Ala
            100                 105                 110

Ala Gly Asp Asn Ser Asp Val Asn Tyr Ile Ile Val Asp Asp Val Ile
        115                 120                 125

Glu Gly Lys Asp Asn Val Ala Ser Val Thr Phe Ala Asp Asn Glu Ala
    130                 135                 140

Ala Tyr Leu Ala Gly Ile Ala Ala Ala Lys Thr Thr Lys Thr Lys Val
145                 150                 155                 160

Val Gly Phe Val Gly Gly Met Glu Gly Thr Val Ile Thr Arg Phe Glu
                165                 170                 175

Lys Gly Phe Glu Ala Gly Val Lys Ser Val Asp Asp Ser Ile Gln Ile
            180                 185                 190

Lys Val Asp Tyr Ala Gly Ser Phe Gly Asp Ala Ala Lys Gly Lys Thr
        195                 200                 205

Ile Ala Ala Ala Gln Tyr Ala Gly Gly Ala Asp Val Ile Tyr Gln Ala
    210                 215                 220

Ala Gly Gly Thr Gly Ala Gly Val Phe Asn Glu Ala Lys Ala Val Asn
225                 230                 235                 240

Glu Lys Lys Asp Glu Ala Asp Lys Val Trp Val Ile Gly Val Asp Arg
                245                 250                 255
 
Asp Gln Lys Glu Glu Gly Lys Tyr Thr Ser Lys Asp Gly Lys Glu Ser
            260                 265                 270

Asn Phe Val Leu Ala Ser Ser Ile Lys Gln Val Gly Lys Ser Val Gln
        275                 280                 285

Leu Ile Asn Lys Leu Val Thr Asp Lys Lys Phe Pro Gly Gly Lys Thr
    290                 295                 300

Thr Val Tyr Gly Leu Lys Asp Gly Gly Val Asp Ile Ala Thr Thr Asn
305                 310                 315                 320

Leu Ser Asp Asp Ala Ile Lys Ala Val Lys Glu Ala Lys Glu Lys Ile
                325                 330                 335

Ile Ser Gly Asp Val Lys Val Pro Glu Lys
            340                 345
<210> 7
   <211> 1149
   <212> DNA
   <213> Artificial Sequence
<220>
   <223> rSUB1868 serine proteinase
<400> 7 
atgggcagca gccatcatca tcatcatcac agcagcggcc tggtgccgcg cggcagccat 60

atgacaaatc ttaataaccc aacgacgaca agtaaagtaa cctataaaaa tactactaat 120

acgactaaag ctgttaaagt gattcaagat gcagttgttt ctgtagttaa ctatcaaaaa 180

aatgattctt taaactcagc catggatatt tttagtcaag gtgattcatc aactaaagag 240

aatgatggac tttctattta tagtgaagga tcaggtgtta tatacaaaaa agatggtgat 300

tctgcatatc tggtaaccaa caaccacgta atagacaaag ctgaaagaat tgaaattatt 360

ttagctgatg gttcaaaagt tgttgggaaa ttaattggtg ctgacactta ttctgacctg 420

gctgttgtaa aaatttcttc agacaaaatt aagactgtag ctcagtttgc agattcttcc 480

aaaataaaca taggtgaagt tgcaattgca attggtagtc ctcttggaac agaatatgct 540

aattccgtaa ctgaaggaat tgtttcaagt ttaagtagaa cagtaacttt aaaaaatgaa 600

gaaggacaaa ctgtttcaac taatgccatt caaacagatg ctgctattaa ccctggaaac 660

tctggcggac ctttaattaa tattgaagga caaattattg gaataaactc tagcaaaatc 720

tcacagtcta aatcatctgg aaatgcagtc gaaggaatgg gatttgcaat tccagctaat 780

gacgttatta aaattattaa ccaacttgaa agcaaaggcg aagtagttcg acctgcatta 840

ggtatttcaa tggttaatct aagtgattta tcaacaaatg cccttgatca gctcaaagtt 900
 
ccaaaaaatg ttactagtgg tatcgtagtt gctaaagtcg tagacaatat gcctgcctca 960

ggaaaacttg aacaatatga tattatcact gaaattgatg gggaagaagt gagcagtaca 1020

agtgatttac aaagtattct gtatgggcat gatattaatg ataccgtaaa agtcactttt 1080

tatagaggta atgataagaa atctactact attgaattaa ctaaaactac caaagattta 1140

gaaaaataa 1149
<210> 8
   <211> 382
   <212> PRT
   <213> Artificial Sequence
<220>
   <223> rSUB1868 serine proteinase
<400> 8

Met Gly Ser Ser His His His His His His Ser Ser Gly Leu Val Pro
1               5                   10                  15

Arg Gly Ser His Met Thr Asn Leu Asn Asn Pro Thr Thr Thr Ser Lys
            20                  25                  30

Val Thr Tyr Lys Asn Thr Thr Asn Thr Thr Lys Ala Val Lys Val Ile
        35                  40                  45

Gln Asp Ala Val Val Ser Val Val Asn Tyr Gln Lys Asn Asp Ser Leu
    50                  55                  60

Asn Ser Ala Met Asp Ile Phe Ser Gln Gly Asp Ser Ser Thr Lys Glu
65                  70                  75                  80

Asn Asp Gly Leu Ser Ile Tyr Ser Glu Gly Ser Gly Val Ile Tyr Lys
                85                  90                  95

Lys Asp Gly Asp Ser Ala Tyr Leu Val Thr Asn Asn His Val Ile Asp
            100                 105                 110

Lys Ala Glu Arg Ile Glu Ile Ile Leu Ala Asp Gly Ser Lys Val Val
        115                 120                 125

Gly Lys Leu Ile Gly Ala Asp Thr Tyr Ser Asp Leu Ala Val Val Lys
    130                 135                 140

Ile Ser Ser Asp Lys Ile Lys Thr Val Ala Gln Phe Ala Asp Ser Ser
145                 150                 155                 160

Lys Ile Asn Ile Gly Glu Val Ala Ile Ala Ile Gly Ser Pro Leu Gly
                165                 170                 175

Thr Glu Tyr Ala Asn Ser Val Thr Glu Gly Ile Val Ser Ser Leu Ser
        180                 185                 190

Arg Thr Val Thr Leu Lys Asn Glu Glu Gly Gln Thr Val Ser Thr Asn
    195                 200                 205

Ala Ile Gln Thr Asp Ala Ala Ile Asn Pro Gly Asn Ser Gly Gly Pro
    210                 215                 220

Leu Ile Asn Ile Glu Gly Gln Ile Ile Gly Ile Asn Ser Ser Lys Ile
225                 230                 235                 240

Ser Gln Ser Lys Ser Ser Gly Asn Ala Val Glu Gly Met Gly Phe Ala
                245                 250                 255

Ile Pro Ala Asn Asp Val Ile Lys Ile Ile Asn Gln Leu Glu Ser Lys
        260                 265                 270

Gly Glu Val Val Arg Pro Ala Leu Gly Ile Ser Met Val Asn Leu Ser
    275                 280                 285

Asp Leu Ser Thr Asn Ala Leu Asp Gln Leu Lys Val Pro Lys Asn Val
    290                 295                 300

Thr Ser Gly Ile Val Val Ala Lys Val Val Asp Asn Met Pro Ala Ser
305                 310                 315                 320

Gly Lys Leu Glu Gln Tyr Asp Ile Ile Thr Glu Ile Asp Gly Glu Glu
                325                 330                 335

Val Ser Ser Thr Ser Asp Leu Gln Ser Ile Leu Tyr Gly His Asp Ile
        340                 345                 350

Asn Asp Thr Val Lys Val Thr Phe Tyr Arg Gly Asn Asp Lys Lys Ser
    355                 360                 365

Thr Thr Ile Glu Leu Thr Lys Thr Thr Lys Asp Leu Glu Lys
    370                 375                 380

## Description

### FIELD OF THE INVENTION

The present invention provides streptococcal antigens capable of raising host immune responses and for use as vaccines to protect against and/or reduce instances of *Streptococcus* infections. Furthermore, the invention provides streptococcal antigens which may find application in the diagnosis of Streptococcal infections and/or diseases and/or the detection or identification of specific streptococcal species.

### BACKGROUND OF THE INVENTION

*Streptococcus uberis* is an important mastitis-causing pathogen, responsible for a large proportion of both clinical and sub-clinical cases of mastitis in many parts of the world. The pathogen affects cattle, goats and sheep; however, infection of cattle is of primary importance due to its impact on the dairy industry, through welfare issues for affected animals, and also because of the significant financial impact on producers. As a species, *S. uberis* is highly-heterogenous; it is biochemically and physiologically ill-defined, and is serologically heterogenous (Hardie, 1986). Furthermore, there is evidence of genetic heterogeneity, driven predominantly through horizontal gene transfer (Coffey *et al.,* 2006). Originally, DNA-DNA hybridization studies suggested the existence of two related *S. uberis* genotypes (designated types I and II) (Garvie & Bramley 1979; Collins *et al.* 1984), both associated with the bovine host and a cause of mastitis. Type II *S*. *uberis* was later re-classified as *Streptococcus parauberis* (Williams and Collins, 1990), which is also increasingly found as a cause of disease in fish; significantly, phylogenetic studies have since shown the two species to be related (Nho *et al.,* 2011), and hence they are likely to share conserved portions of genome. An immunogenic composition comprising an *S. uberis* protein is disclosed in WO 2010/041056.

It is, in part, due to the heterogeneity of the *S. uberis* population that efforts to develop an effective vaccine have been unsuccessful. This is because *S. uberis* antigens which have been shown to have promise as vaccines have not always been found to be conserved amongst the broader population.

It is an object of the present invention to provide antigens which are conserved among strains of *S. uberis* and which may have use or be exploited, in vaccines and methods of diagnosis, detection and/or identification.

### SUMMARY OF THE INVENTION

The present invention is based upon the identification of a number of antigens derived from a species of the genus *Streptococcus,* which can be used to raise immune responses in animals - particularly those animals susceptible or predisposed to infection by (or with) the *Streptococcus* species. The invention provides the composition defined in claims 1-3.

The antigens provided by this invention may be exploited to provide compositions and vaccines for raising protective immune responses in animals - the protective immune responses serving to reduce, prevent, treat or eliminate certain *Streptococcus* infections as well as diseases and/or conditions caused or contributed to thereby.

Disclosed herein are one or more *Streptococcus uberis* antigen(s) or a fragment or fragments thereof, for use in raising an immune response in an animal.

*Streptococcus uberis* is a Gram-positive non-motile coccus. It can infect the bovine mammary gland where it can cause mastitis, an inflammatory disease - indeed, infection with *S. uberis* is one of the major causes of bovine mastitis worldwide. *S.uberis* can be detected in faeces and isolated from environments (for example pasture and/or bedding materials) populated by cattle. It has been placed within the pyogenic cluster, a large grouping containing the human pathogens *Streptococcus pyogenes,* the zoonotic *Streptococcus agalactiae* and a number of animal pathogens occupying diverse ecological niches including *Streptococcus equi* and *Streptococcus canis.*

*Streptococcus uberis* is a highly-heterogeneous species but despite this, the inventors have discovered antigens which are conserved in different *S. uberis* strains. That is to say, while the antigens of this invention may be derived or obtained from a single *S. uberis* strain, they may protect animals against infections and/or diseases caused or contributed to different (heterologous) *Streptococcus uberis* strains. Thus the antigens disclosed herein may be used to facilitate the detection or identification of one, two or more different *S. uberis* strains. Furthermore, compositions of this invention may be used to raise immune responses which are protective against infections, diseases and/or conditions caused by any one or more of a number of different *S. uberis* strains. The inventors have noted that the antigens provided by this invention may also be used to protect animals against infections (or diseases) caused or contributed to by other *Streptococcus* sp., including, for example, infections, diseases and/or conditions caused or contributed to by (but not limited to) *Streptocuccus parauberis, S. agalactiae* and *Streptococcus dysgalactiae.*

The antigens described herein may find application in the detection, identification and/or diagnosis of infections, diseases and/or conditions with a Streptococcal (including, but not limited to, *S. uberis, S. parauberis, S. agalactiae* and *S. dysgalactiae*) aetiology.

For convenience, each of the specific *Streptococcus* species and strains relevant to this invention shall be collectively referred to under the general term *"Streptococcus"* or "streptococcal". Moreover, it should be understood that references to *S. uberis* include all related *S. uberis* strains and variants.

An immune response which protects against infection by/with a pathogen or against certain diseases or conditions, may be referred to as a 'protective response'. Therefore, in the context of this invention, the immune responses elicited by the antigens described herein may be regarded as 'protective' immune responses.

The antigens provided by this invention are immunogenic or antigenic in that they elicit host immune responses; the precise nature of the response (humoral and/or cellular for example) may depend on the formulation of the antigen, its route of administration and/or the presence or absence of adjuvant.

The effectiveness of any immune response elicited by the antigens of this invention may be assessed relative to the prevalence or rate of the relevant *Streptococcus* based infection/disease among a population of animals not exposed to, contacted with or administered antigens of this invention. One of skill will appreciate that animals not exposed to, contacted with or administered an antigen of this invention may lack a protective immune response and are therefore more susceptible to *Streptococcus* infections and/or diseases.

Disclosed herein is a composition, immunogenic composition or vaccine composition comprising one or more of the *Streptococcus uberis* antigens described herein. The various compositions may be for use in raising an immune response in an animal. In one embodiment, the immune response is a protective response.

Also described is the use of one or more *Streptococcus uberis* antigens or a fragment(s) thereof for the manufacture of a medicament or vaccine for use in the treatment and/or prevention of a *Streptococcus uberis* infection and/or a disease or condition caused thereby or associated therewith.

There is also described a method of raising an anti-*Streptococcus uberis* immune response in an animal, said method comprising the step of administering to an animal, an amount of one or more *Streptococcus* antigen(s) or fragment(s) thereof, sufficient to induce an anti-*Streptococcus uberis* immune response.

The term "animal" encompasses any animal known to be susceptible to a *Streptococcus* infection, disease or condition. For example, any animal susceptible to infections, diseases or conditions caused or contributed to by *S. uberis, S. parauberis, S. agalactiae* and/or *S. dysgalactiae* (or indeed any strains of any of these) is/are encompassed under the general term "animal" as used herein. For example, the term "animal" may include humans and animals collectively known as avian (birds), piscine (e.g. fish), porcine (pig), bovine (e.g. cattle), caprine (e.g. goats) and/or ovine (e.g. sheep) animals. As such, there is described antigens and compositions for use in raising immune responses in human, avian, piscine, caprine, bovine, porcine and/or ovine subjects such as, for example, fish, cattle, sheep and goats.

Diseases and/or conditions caused or contributed to by *Streptococcus (including for example S. uberis* and *S. dysgalactiae*) include clinical and sub-clinical cases of mastitis. As such, the antigens provided by this invention may be used to raise immune responses in animals which are susceptible, predisposed and/or prone to developing (streptococcal based) mastitis, said responses being protective against the development of mastitis. For example, the invention may be used to raise immune responses in human, porcine, bovine, caprine and/or ovine animals, said responses being protective against the development of mastitis.

In view of the above, there is disclosed:
(i) *Streptococcus uberis* antigens and compositions, vaccines and medicaments comprising the same; and
(ii) methods exploiting one or more antigens derived from *Streptococcus uberis;*
for use in raising immune responses in human, avian, piscine, bovine, porcine, caprine and/or ovine animals.

Furthermore, there is disclosed:
(i) Streptococcus uberis antigens and compositions, vaccines and medicaments comprising the same; and
(ii) methods exploiting one or more antigens derived from Streptococcus uberis;
for use in treating mastitis; and/or
for use in raising immune responses in human, avian, piscine, bovine, porcine, caprine and/or ovine animals, said responses being protective against the development of mastitis.

It should be understood that all references to "antigen" encompass immunogenic components, proteins or peptides derived from *Streptococcus uberis.* The antigens may comprise cell-surface antigens and intracellular antigens. The antigens may be prepared using recombinant technology (as described later) but may be obtained or purified from *S. uberis* cells and/or cell cultures. For example, *S. uberis* cell-surface antigens may be isolated from *S. uberis* cell-wall preparations.

Specifically, the term "antigen" encompasses the exemplary *Streptococcus uberis* antigens listed as (i)-(xvii) in Table 1 below:

**Table 1**

| | **Locus tag** | **Protein** |
|---|---|---|
| i | **SUB0423** | ferrichrome binding protein |
| ii | **SUB0604** | elongation factor Tu |
| iii | **SUB0950** | Lipoprotein |
| iv | **SUB1868** | serine protease |

The *S. uberis* antigens identified in Table 1 are those identified in each of the cell-wall sub-cellular fractions of a number of *S. uberis* strains (as determined by mass spectrometry-based proteomic analysis). Consequently, these antigens may be exploited and used in the methods, vaccines and/or compositions described herein. Indeed the inventors have noted that a vaccine based on a combination of all four of the antigens disclosed in Table 1 is particularly effective at raising immune responses in cattle which are protective against infection and/or diseases caused or contributed to by *S. uberis,* including, for example, mastitis.

The target genes of the *S. uberis* antigens shown in Table 1 are shown in Table 2 below.

**Table 2**

| **Target gene*** |
|---|
| **SUB0423** |
| ferrichrome binding protein |
| YP_002561776 |

| **SUB0604** |
|---|
| elongation factor Tu |
| YP_002561947 |

| **SUB0950** |
|---|
| lipoprotein |
| YP_002562276 |

| **SUB1868** |
|---|
| serine proteinase |
| YP_002563137 |

Table 2a shows Conserved carriage of *Streptococcus uberis* antigen-encoding genes.

| | | | **Gene carriage** | | | |
|---|---|---|---|---|---|---|
| **Strain identification** | **Country of origin** | **Species of origin** | **sub0423** | **sub0604** | **sub0950** | **Sub1868** |
| T1-4 | UK | Bovine | + | + | + | + |
| T1-5 | UK | Bovine | + | + | + | + |
| T1-6 | UK | Bovine | + | + | + | + |
| T1-9 | UK | Bovine | + | + | + | + |
| T1-10 | UK | Bovine | + | + | + | + |
| T1-20 | UK | Bovine | + | + | + | + |
| T1-21 | UK | Bovine | + | + | + | + |
| T1-22 | UK | Bovine | + | + | + | + |
| T1-36 | UK | Bovine | + | + | + | + |
| T1-37 | UK | Bovine | + | + | + | + |
| T1-38 | UK | Bovine | + | + | + | + |
| T1-43 | UK | Bovine | + | + | + | + |
| T1-44 | UK | Bovine | + | + | + | + |
| T1-45 | UK | Bovine | + | + | + | + |
| T1-79 | UK | Bovine | + | + | + | + |
| T1-80 | UK | Bovine | + | + | + | + |
| T1-81 | UK | Bovine | + | + | + | + |
| T2-1 | UK | Bovine | + | + | + | + |
| T2-2 | UK | Bovine | + | + | + | + |
| T2-3 | UK | Bovine | + | + | + | + |
| T2-4 | UK | Bovine | + | + | + | + |
| T2-5 | UK | Bovine | + | + | + | + |
| T2-6 | UK | Bovine | + | + | + | + |
| T2-7 | UK | Bovine | + | + | + | + |
| T2-8 | UK | Bovine | + | + | + | + |
| T2-18 | UK | Bovine | + | + | + | + |
| T2-19 | UK | Bovine | + | + | + | + |
| T2-35 | UK | Bovine | + | + | + | + |
| T2-36 | UK | Bovine | + | + | + | + |
| T2-37 | UK | Bovine | + | + | + | + |
| T2-53 | UK | Bovine | + | + | + | + |
| T2-54 | UK | Bovine | + | + | + | + |
| T2-55 | UK | Bovine | + | + | + | + |
| T2-63 | UK | Bovine | + | + | + | + |
| T2-73 | UK | Bovine | + | + | + | + |
| I2 | Italy | Ovine | + | + | + | + |
| I3 | Italy | Ovine | + | + | + | + |
| I4 | Italy | Ovine | + | + | + | + |
| I5 | Italy | Ovine | + | + | + | + |
| I6 | Italy | Ovine | + | + | + | + |
| I7 | Italy | Ovine | + | + | + | + |
| I8 | Italy | Ovine | + | + | + | + |
| I10 | Italy | Ovine | + | + | + | + |
| I11 | Italy | Ovine | + | + | + | + |
| I13 | Italy | Ovine | + | + | + | + |
| I14 | Italy | Ovine | + | + | + | + |
| I23 | Italy | Bovine | + | + | + | + |
| I24 | Italy | Bovine | + | + | + | + |
| I25 | Italy | Bovine | + | + | + | + |
| I26 | Italy | Bovine | + | + | + | + |
| I34 | Italy | Bovine | + | + | + | + |
| I38 | Italy | Bovine | + | + | + | + |
| I44 | Italy | Bovine | + | + | + | + |
| I45 | Italy | Bovine | + | + | + | + |
| I46 | Italy | Bovine | + | + | + | + |
| I47 | Italy | Bovine | + | + | + | + |
| I48 | Italy | Bovine | + | + | + | + |
| I50 | Italy | Bovine | + | + | + | + |
| 1;1 | USA | Bovine | + | + | + | + |
| 1;3 | USA | Bovine | + | + | + | + |
| 1;4 | USA | Bovine | + | + | + | + |
| 1;40 | USA | Bovine | + | + | + | + |
| 1;41 | USA | Bovine | + | + | + | + |
| 2;13 | USA | Bovine | + | + | + | + |
| 2;14 | USA | Bovine | + | + | + | + |
| 2;15 | USA | Bovine | + | + | + | + |
| 2;21 | USA | Bovine | + | + | + | + |
| 2;22 | USA | Bovine | + | + | + | + |
| 2;100 | USA | Bovine | + | + | + | + |
| T1-7 (control) | UK | Bovine | - | - | - | - |

| | | | | | | |
|---|---|---|---|---|---|---|
| ^{†}The genes encoding *S. uberis* proteins previously found to be conserved by mass spectrometric analyses were assessed for carriage, by PCR, among a diverse panel of strains. The nomenclature of the conserved genes is derived from the equivalent open reading frames in the published *S. uberis* 0140J genome, and is as follows: sub0423, ferrichrome-binding protein (accession #YP_002561776); sub0604, elongation factor Tu (accession #YP_002561947); sub0950, lipoprotein (accession #YP_002562276); sub1869, serine protease (accession #YP_002563137). All four genes were found to be present in all *S. uberis* strains, with the exception of sub0604 in 1 strain (1;42) from the USA. ^{‡}An *Enterococcus* spp. strain (T1-7), isolated from a case of bovine mastitis in the UK, was included as a control, and none of the target genes were found to be present. | | | | | | |

In view of the above, the term "antigen" as used herein, encompasses antigens encoded by or comprising/consisting (essentially of) the sequences deposited under each of the accession numbers identified in Table 2. It should be noted that the sequences deposited under each of the accession numbers identified above are derived from a *S. uberis* strain designated 0140J (accession number NC 012044). Thus, while the invention encompasses antigens derived from this *S. uberis* strain, it also encompasses the identical, homologous or othologous antigens present in other *Streptococci* and/or *S. uberis* strains.

The inventors have recombinantly prepared the *S. uberis* antigens and the sequences of the cloned antigens are provided in Table 3 below. One of skill will appreciate that recombinant sequences may comprise sequences which differ from any corresponding wild type or reference sequences and may comprise, for example, sequences which encode protein or peptide tags. Recombinant sequences may be modified by, for example, the deletion of signal peptide sequences. The sequences presented in Table 3 have a 5'-nucleotide sequence encoding a 6 x histidine tag - a tag of this type may be used for purification purposes. Also, some of the sequences presented in Table 3 have been further modified to lack sequences encoding secretion signal peptides present in the corresponding wild type sequences.

**Table 3**

| **Recombinant gene sequence** | **Translated product** |
|---|---|
| **rSUB0423** ferrichrome binding protein (SEQ ID NO: 1 and 2) | |
| Seq 1 | Seq 2 |
| ATGGGCAGCAGCCATCATCATCATCATCACAGCAGCGGCCTGGTGCCGCGC GGCAGCCATA TGCTCGAGATGTCACAAAGCACAAAGCAAGAAGATCATAAAACAAAACTAT CACAAATGC CAAAGATCTCTGGTTTTACCTATAAAGGGAAGGTACCAGAAAACCCTAAAA GAGTAGTTA GTTTATCTTCAACCTACACCGGTTATTTGGCAAAGCTCGATATCCCACTAG TTGGAATCA CTTCTTATGATCACAAAAATCCCGTCTTAAAGAAATACATCAAGGATGCTA AAGTTGTCT CTGCAACCGACCTAGAAAGCATTACGGCCTTGGAACCTGATTTAATTATTG TGGGTTCAA ATGAAGAAAATATCAGTCAATTAGCTGAAATCGCTCCCCTTATTTCCATTG AATACCGCA AACATGACTATTTACAGGTATTCTCAGATTTTGGTAAAGTCTTTAACAAAA CCAAAGAAA CCGACAAATGGTTACAGGAATGGAAAACAAAAACAGCTTCTTTTGAAAGTG ACGTTAAAG CAGTTACAGGTAATAATGCTACCTTTACCATAATGGGATTATATGAGAAAG ATATCTATC TTTTCGGTAAAGATTGGGGTCGTGGTGGTGAAATCATTCACCAAGCCTTCC AATATCAAG CTCCAGAAAAAGTAAAAATGGAGGTTTTCCCAAAAGGCTATTTGTCCATTT CACAAGAAG TTCTTCCAGATTATATATTGGTGATTATGTCGTTGTCGCTGCAGAGGATGAAA AAACAGGTT CTTCTCTTTATGAAAGTGACCTTTGGAAAAATATACCAGCCGTTCAAAAAA ATCATGTCA TAAATGTTAATGCGAATACCTTTTATTTCACTGACCCTCTGTCATTAGAGT ATGAATTAA AAACCTTAACGGATGCTATCTTGACTCAGAAAACTCACAACTA | MGSSHHHHHHSSGLVPRGSHMLEMSQSTKQEDHKTKLSQMPKISGFTYKGK VPENPKRVV SLSSTYTGYLAKLDIPLVGITSYDHKNPVLKKYIKDAKVVSATDLESITAL EPDLIIVGSN EENISQLAEIAPLISIEYRKHDYLQVFSDFGKVFNKTKETDKWLQEWKTKT ASFESDVKA VTGNNATFTIMGLYEKDIYLFGKDWGRGGEIIHQAFQYQAPEKVKMEVFPK GYLSISQEV LPDYIGDYVVVAAEDEKTGSSLYESDLWKNIPAVQKNHVINVNANTFYFTD PLSLEYELK TLTDAILTQKTHN |

| **rSUB0604** elongation factor Tu (SEQ ID NO: 3 and 4) | |
|---|---|
| Seq 3 | Seq 4 |
| TGGGCAGCAGCCATCATCATCATCATCACAGCAGCGGCCTGGTGCCGCGCG GCAGCCATA TGCTCGAGATGGCAAAAGAAAAATACGATCGTAGTAAACCCCACGTTAACA TTGGTACAA TTGGACACGTTGACCACGGTAAAACTACTTTGACAGCTGCAATTACAACTG TACTTGCTC GTCGCTTACCAACTTCAGTTAACCAACCAAAAGATTACGCTTCTATCGATG CTGCTCCAG AAGAGCGCGAACGCGGAATCACTATCAACACTGCACACGTTGAGTACGAAA | MGSSHHHHHHSSGLVPRGSHMLEMAKEKYDRSKPHVNIGTIGHVDHGKTTL TAAITTVLA RRLPTSVNQPKDYASIDAAPEERERGITINTAHVEYETETRHYAHIDAPGH ADYVKNMIT GAAQMDGAILVVASTDGPMPQTREHILLSRQVGVKHLIVFMNKIDLVDDEE LLELVEMEI RDLLSEYDFPGDDLPVIQGSALKALEGDSKYEDIIMELMKTADEYIPEPER DTDKPLLLP |
| CTGAAACTC GTCACTATGCCCACATTGATGCCCCAGGACACGCGGACTATGTTAAAAACA TGATCACTG GTGCTGCCCAAATGGACGGAGCTATCCTTGTTGTTGCATCAACTGATGGAC CAATGCCAC AAACTCGTGAGCACATCCTTCTTTCACGCCAAGTTGGTGTTAAACACCTTA TCGTTTTCA TGAACAAAATCGACCTTGTTGACGATGAAGAATTGCTTGAATTAGTTGAAA TGGAAATCC GTGACCTTCTTTCAGAATACGATTTCCCAGGTGATGACCTACCAGTTATCC AAGGTTCAG CTCTTAAAGCTCTTGAAGGTGATTCTAAATACGAAGACATCATCATGGAAT TGATGAAAA CTGCTGATGAGTATATTCCAGAACCAGAACGTGATACAGACAAACCATTAC TTCTTCCAG TCGAAGACGTATTCTCAATCACAGGTCGTGGTACTGTAGCTTCAGGACGTA TCGATCGTG GTACTGTTCGTGTCAACGACGAAATTGAAATCGTTGGTATCAAAGAAGAAA CTAAAAAAG CAGTTGTTACTGGTGTTGAAATGTTCCGTAAACAACTTGACGAAGGTCTTG CAGGAGATA ACGTAGGTATCCTTCTTCGTGGTGTTCAACGTGACGAAATCGAACGTGGAC AAGTTATTG CTAAACCAGGTTCAATCAACCCACACACTAAATTCAAAGGTGAAGTTTACA TCCTTTCTA AAGATGAAGGTGGACGTCATACTCCATTCTTCAACAACTACCGTCCTCAAT TCTATTTCC GTACAACTGACGTAACAGGTTCAATCGAACTTCCAGCTGGTACTGAAATGG TAATGCCTG GTGATAACGTGACAATCAGCGTTGAGTTGATCCACCCAATCGCCGTTGAAC AAGGTACTA CTTTCTCAATCCGTGAAGGTGGACGTACTGTTGGTTCAGGTATTGTTTCAG AAATCGAAG CTTAA | VEDVFSITGRGTVASGRIDRGTVRVNDEIEIVGIKEETKKAVVTGVEMFRK QLDEGLAGD NVGILLRGVQRDEIERGQVIAKPGSINPHTKFKGEVYILSKDEGGRHTPFF NNYRPQFYF RTTDVTGSIELPAGTEMVMPGDNVTISVELIHPIAVEQGTTFSIREGGRTV GSGIVSEIE A |

| **rSUB0950** lipoprotein (SEQ ID NO: 5 and 6) | |
|---|---|
| Seq 5 | Seq 6 |
| ATGGGCAGCAGCCATCATCATCATCATCACAGCAGCGGCCTGGTGCCGCGC GGCAGCCAT ATGCTCGAGATGGATAGCAAAGATGCTAAAACAGATTTAAAAGCTGCTATT GTTACTGAT ACAGGTGGTGTTGATGATAAATCATTTAACCAATCTGCTTGGGAAGGTTTA GAAGCTTGG GGTAAAGAAAATGGGCTTAAAAAAGGTGCTGGTTTCGACTACTTCCAATCA AATAGTGAA TCAGAATATGCTACTAATCTTGACACTGCTGTCTCAAGTGGTTATAACGTA GTATATGGA ATCGGATTTGCCCTTAAAGATGCAATTGATAAAGCTGCTGGTGACAATAGT GATGTTAAC | MGSSHHHHHHSSGLVPRGSHMLEMDSKDAKTDLKAAIVTDTGGVDDKSFNQ SAWEGLEA WGKENGLKKGAGFDYFQSNSESEYATNLDTAVSSGYNVVYGIGFALKDAID KAAGDNSDVN YIIVDDVIEGKDNVASVTFADNEAAYLAGIAAAKTTKTKVVGFVGGMEGTV ITRFEKGFE AGVKSVDDSIQIKVDYAGSFGDAAKGKTIAAAQYAGGADVIYQAAGGTGAG VFNEAKAVN EKKDEADKVWVIGVDRDQKEEGKYTSKDGKESNFVLASSIKQVGKSVQLIN KLVTDKKFP |
| TATATTATCGTTGACGATGTCATCGAAGGAAAAGATAATGTTGCAAGTGTA ACTTTTGCG GATAACGAAGCTGCTTATCTTGCTGGTATTGCTGCAGCTAAAACTACAAAA ACTAAAGTA GTAGGTTTTGTAGGTGGTATGGAAGGTACTGTTATCACTCGTTTTGAAAAA GGTTTTGAG GCGGGAGTGAAATCAGTTGATGATTCTATCCAAATCAAAGTTGACTACGCT GGATCATTT GGTGATGCTGCTAAAGGTAAAACAATTGCCGCAGCTCAATATGCAGGTGGT GCTGACGTT ATTTATCAAGCCGCTGGTGGTACTGGAGCAGGTGTCTTCAATGAAGCTAAA GCTGTAAAT GAGAAAAAAGATGAAGCTGATAAAGTTTGGGTAATCGGTGTAGACCGTGAC CAAAAAGAG GAAGGTAAATACACTTCAAAAGACGGTAAAGAATCTAACTTTGTTCTAGCA TCTTCAATT AAACAAGTTGGTAAATCTGTACAACTGATTAACAAACTTGTTACTGATAAA AAATTCCCT GGTGGAAAAACAACTGTTTATGGATTAAAAGATGGTGGTGTTGATATTGCA ACAACAAAC CTTTCTGATGATGCTATAAAAGCTGTTAAAGAAGCTAAAGAAAAAATTATT TCTGGCGAT GTAAAAGTTCCTGAAAAATAA | GGKTTVYGLKDGGVDIATTNLSDDAIKAVKEAKEKIISGDVKVPEK |

| **rSUB1868** serine proteinase (SEQ ID NO: 7 and 8) | |
|---|---|
| Seq 7 | Seq 8 |
| ATGGGCAGCAGCCATCATCATCATCATCACAGCAGCGGCCTGGTGCCGCGC GGCAGCCAT ATGACAAATCTTAATAACCCAACGACGACAAGTAAAGTAACCTATAAAAAT ACTACTAAT ACGACTAAAGCTGTTAAAGTGATTCAAGATGCAGTTGTTTCTGTAGTTAAC TATCAAAAA AATGATTCTTTAAACTCAGCCATGGATATTTTTAGTCAAGGTGATTCATCA ACTAAAGAG AATGATGGACTTTCTATTTATAGTGAAGGATCAGGTGTTATATACAAAAAA GATGGTGAT TCTGCATATCTGGTAACCAACAACCACGTAATAGACAAAGCTGAAAGAATT GAAATTATT TTAGCTGATGGTTCAAAAGTTGTTGGGAAATTAATTGGTGCTGACACTTAT TCTGACCTG GCTGTTGTAAAAATTTCTTCAGACAAAATTAAGACTGTAGCTCAGTTTGCA GATTCTTCC AAAATAAACATAGGTGAAGTTGCAATTGCAATTGGTAGTCCTCTTGGAACA GAATATGCT AATTCCGTAACTGAAGGAATTGTTTCAAGTTTAAGTAGAACAGTAACTTTA AAAAATGAA GAAGGACAAACTGTTTCAACTAATGCCATTCAAACAGATGCTGCTATTAAC CCTGGAAAC TCTGGCGGACCTTTAATTAATATTGAAGGACAAATTATTGGAATAAACTCT | MGSSHHHHHHSSGLVPRGSHMTNLNNPTTTSKVTYKNTTNTTKAVKVIQDA VVSVVNYQK NDSLNSAMDIFSQGDSSTKENDGLSIYSEGSGVIYKKDGDSAYLVTNNHVI DKAERIEII LADGSKVVGKLIGADTYSDLAVVKISSDKIKTVAQFADSSKINIGEVAIAI GSPLGTEYA NSVTEGIVSSLSRTVTLKNEEGQTVSTNAIQTDAAINPGNSGGPLINIEGQ IIGINSSKI SQSKSSGNAVEGMGFAIPANDVIKIINQLESKGEVVRPALGISMVNLSDLS TNALDQLKV PKNVTSGIVVAKVVDNMPASGKLEQYDIITEIDGEEVSSTSDLQSILYGHD INDTVKVTF YRGNDKKSTTIELTKTTKDLEK |
| AGCAAAATC TCACAGTCTAAATCATCTGGAAATGCAGTCGAAGGAATGGGATTTGCAATT CCAGCTAAT GACGTTATTAAAATTATTAACCAACTTGAAAGCAAAGGCGAAGTAGTTCGA CCTGCATTA GGTATTTCAATGGTTAATCTAAGTGATTTATCAACAAATGCCCTTGATCAG CTCAAAGTT CCAAAAAATGTTACTAGTGGTATCGTAGTTGCTAAAGTCGTAGACAATATG CCTGCCTCA GGAAAACTTGAACAATATGATATTATCACTGAAATTGATGGGGAAGAAGTG AGCAGTACA AGTGATTTACAAAGTATTCTGTATGGGCATGATATTAATGATACCGTAAAA GTCACTTTT TATAGAGGTAATGATAAGAAATCTACTACTATTGAATTAACTAA AACTACC AAAGATTTAGAAAAATAA | |

Disclosed herein are one or more recombinant forms of any of the *S. uberis* antigens identified in this specification - the recombinant sequences being optionally modified (relative to the corresponding wild type sequence) to include sequences encoding tagging or labelling moieties and or through the deletion of one or more wild type sequences or domains - such as, for example, a sequences or domains encoding a signal peptide.

Also described are the equivalent or corresponding antigens present in other *Streptococcal* species, including for example the identical, homologous or orthologous antigens present in *S. agalactiae, S. parauberis* and *S. dysgalactiae.* One of skill will appreciate that sequences derived from strains other strains of S. *uberis* may differ in nucleic acid sequence. These sequence differences may arise as a result of the natural variation, mutation(s) and/or (single or multiple) nucleotide polymorphisms that often exist between the genomes of related species. Where the biological function of a particular protein is reliant on a particular amino acid sequence, selective pressure will tend to ensure that only mutations which introduce silent or conservative changes to the encoded protein are retained within the microbial population. Consequently, one of skill will recognise that despite divergence in gene coding sequences, retention of biological function through retention of functional epitopes may occur, even though the sequence of less-important regions of a protein coding sequence may vary greatly between strains within a species and between species.

The *S. uberis* antigens described may be used to immunise animals in order to raise an immune response that neutralises and/or interferes with/abrogates the function of wild-type proteins produced by *S. uberis* during infection, and in so doing reduce the infective capacity of the pathogen. It therefore follows that the same antigens, or derivatives thereof, may also be used to induce equivalent protective immune responses against pathogens other than *S. uberis.* Indeed, an immune response raised by any of the antigens described herein may offer protection against any pathogen (in particular any other *Streptococcus* species) expressing those antigens.

As is well known in the art, the degeneracy of the genetic code permits substitution of one or more bases in a codon without changing the primary amino acid sequence. Consequently, although the nucleic acid sequences described in this application are known to encode *S. uberis* antigens which elicit immune responses in animals, the degeneracy of the code may be exploited to yield variant nucleic acid sequences which encode the same or similar primary amino acid sequences. Additionally or alternatively, there are described sequences which have been codon optimised, perhaps for expression in certain cellular (for example bacterial) systems. As such, the term "antigen" encompasses nucleic acid sequences which encode the amino acid sequences of the *S. uberis* antigens described herein including for example described in Tables 1 and 2. Also disclosed herein is cDNA generated from messenger RNA encoding any of the antigens of this invention.

It should be understood that this disclosure provides fragments or portions of the various antigens and sequences disclosed herein. The fragments and/or portions of these antigens and/or sequences may themselves provide or encode antigens which are antigenically/immunogenically similar to the complete or whole *Streptococcus uberis* antigens disclosed herein. Thus the fragments and/or portions are (at least immunologically) functional in that they are capable of eliciting an immune response which is substantially identical, or similar, to an immune response elicited by the complete antigen from which the fragment is derived. The terms "fragments" and "portions" as applied to the *S. uberis* antigens of this invention, encompass immunogenic and/or antigenic fragments and/or portions, which fragments and/or portions can be used to raise immune responses in animals. Fragments or portions of any of the antigens disclosed herein may elicit protective immune responses in animals and may comprise epitopes capable of eliciting protective immune responses.

A fragment or portion of an antigen provided herein may comprise any number of amino acid residues or be encoded by any number of nucleic acid residues. For example, fragments of this invention may encompass from about 5 to about 10 residues to about n-1 residues, wherein "n" is the total number of (amino acid or nucleic acid) residues of a *S. uberis* antigen (or antigen coding sequence) described herein. For example, a fragment or portion of a *S. uberis* antigen may comprise or be encoded by a sequence comprising at least about 10, 15, 20, 25, 30, 35, 40, 45, 50, 75, 100, 150, 200, 250, 300 residues - the upper limit (n-1) depending upon the size (n) of the nucleic acid encoding the complete antigen or the number (n) of amino acid residues comprising the primary sequence of the antigen.

In view of the above, the antigen fragments or portions may include fragments and/or portions of any of the sequences identified in Tables 1-3 above.

The term "antigen" may further encompass antigens which exhibit a degree of identity and/or homology to the antigens and/or antigen sequences described herein. By way of example, a homologous or identical sequence provided by this invention may exhibit at least 30%, 35%, 40%, 45%, 50%, 55%, 60%, 65%, 70%, 75%, 80%, 85%, 90%, 95%, 96%, 97%, 98%, 99% homology or identity to the various sequences provided herein - including, for example, any of those sequences identified by the accession numbers in Table 2.

The degree of (or percentage) "homology" between two or more (amino acid or nucleic acid) sequences may be determined by aligning the sequences and determining the number of aligned residues which are identical or which are not identical but which differ by redundant nucleotide substitutions (the redundant nucleotide substitution having no effect upon the amino acid encoded by a particular codon, or conservative amino acid substitutions). Homology may assessed by using the Basic Local Alignment Search Tool (BLAST) (Altschul *et al.,* 1990).

A degree (or percentage) "identity" between two or more (amino acid or nucleic acid) sequences may also be determined by aligning the sequences and ascertaining the number of exact residue matches between the aligned sequences and dividing this number by the number of total residues compared - multiplying the resultant figure by 100 would yield the percentage identity between the sequences. The skilled person will be familiar with the various online alignment tools which can be used to determine the degree or percentage identity between two or more amino acid/nucleic acid sequences. For example, the following sites offer suitable alignment tools: http://www.ebi.ac.uk/Tools/psa/ and http://www.ebi.ac.uk/Tools/msa/clustalw2/

As with the antigenic fragments and/or portions provided herein, any antigens encoded by or comprising/consisting (essentially of) sequences which exhibit homology and/or identity to the sequences described in this application may be immunogenic and suitable for raising immune responses in animals, wherein the immune responses neutralise one or more *S. uberis* antigens and/or are protective against *S. uberis* infection and/or diseases and/or conditions caused or contributed to thereby.

A variant, derivative or mutant antigen may comprise or be encoded by, a nucleic acid or amino acid sequence which comprises one or more nucleotide and/or amino acid substitutions, inversions, additions and/or deletions relative to a reference sequence. A reference sequence may be any of the sequences described in this application. The term "substitution" may encompass one or more conservative substitution(s). One of skill in this field will understand that the term "conservative substitution" is intended to embrace the act of replacing one or more amino acids of a protein or peptide with an alternate amino acid with similar properties and which does not substantially alter the physico-chemical properties and/or structure or function of the native (or wild-type) protein.

Examples of such conservative substitutions are presented in Table 4.

**Table 4. Conservative amino acid substitutions**

| **Residue** | **Abbreviation** | **Conservative substitutions** |
|---|---|---|
| Alanine | Ala | Ser |
| Arginine | Arg | Lys |
| Asparagine | Asn | Gln, His |
| Aspartic acid | Asp | Glu |
| Cysteine | Cys | Asn |
| Glutamic adic | Glu | Ser |
| Glutamine | Gln | Asp |
| Glycine | Gly | Pro |
| Histidine | His | Asn, Gln |
| Isoleucine | Ile | Leu, Val |
| Leucine | Leu | Ile, Val |
| Lysine | Lys | Arg, Gln |
| Methionine | Met | Leu, Ile |
| Phenylalanine | Phe | Met, Leu, Tyr |
| Proline | Pro | |
| Serine | Ser | Thr, Gly |
| Threonine | Thr | Ser, Val |
| Tryptophan | Trp | Tyr |
| Tyrosine | Tyr | Trp, Phe |
| Valine | Val | Ile, Leu |

One of skill will appreciate that the antigens described herein may comprise domains and regions (or epitopes) which represent the immunogenic or active parts. The immunogenic of active parts of an antigen of this invention are those domains, regions or epitopes which are capable of inducing an immune response in the relevant animal. Therefore, antigens may comprise synthetic or recombinant constructs or fusions which comprise these immunogenic or active regions, domains or epitopes. For example, antigens may comprise sequences which exhibit at least 30%, 35%, 40%, 45%, 50%, 55%, 60%, 65%, 70%, 75%, 80%, 85%, 90%, 95%, 96%, 97%, 98%, 99% or 100% homology or identity (as defined above) to the sequences of the active or immunogenic (epitope containing) domains or regions of the antigens described herein, including, for example, those sequences identified by accession number in Table 2.

In the context of this disclosure, a variant, derivative or mutant *S. uberis* antigen may comprise or be encoded by a variant, derivative or mutant sequence which, when compared to a reference sequence (such as for example a wild-type *S. uberis* sequence or a sequence of or encoding any of the specific *S. uberis* antigens presented above, is found to contain one or more amino acid/nucleotide substitutions, additions, deletions and/or inversions.

An antigen which may be regarded as a derivative of the *S. uberis* antigens described herein may further comprise one or more features (for example epitopes or domains) of an *S. uberis* antigen fragment or mutant, variant or derivative described herein, optionally in combination with one or more modifications to the structure of the antigen or one or more of the amino acid residues thereof.

The mutant, variant and/or derivative sequences described herein may be immunogenic and suitable for raising immune responses in animals, wherein the immune responses neutralise one or more *S. uberis* antigens and/or are protective against *S. uberis* infection and/or diseases and/or conditions caused or contributed to thereby.

In view of the above, the present disclosure relates to:
(a) *S. uberis:*
   (i) lipoprotein (acc No: YP_002562276)
   (ii) serine proteinase (acc No: YP_002563137)
   (iiii) ferrichrome binding protein: (acc No: YP_002561776)
   (iv) elongation factor Tu: (acc No: YP_002561947) antigens or (b) proteins having or encoded by sequences comprising, consisting or consisting essentially of sequences which exhibit at least about 60% identity to the sequences described in this application (that is, for example, the sequences disclosed by reference to deposited accession numbers) and/or as (i) - (iv) above;
(c) antigens or proteins having or encoded by or comprising, consisting or consisting essentially of sequences which represent variant , derivative or mutant sequences of those sequences encoding the *S. uberis* antigens described herein and/or as (i) - (iv) above ; and
(d) antigens or proteins having or comprising sequences corresponding to the immunogenic domains of the antigens described herein (for example, the antigens presented in Table 2) and/or as (i) - (iv) above.

Antigens of this type may comprise sequences which share about 60% to about 100% sequence identity with the immunogenic domains of any of these antigens.

It will be appreciated that the *S. uberis* antigens described in this application may be obtained by direct purification from *S. uberis* cultures and/or protein/membrane preparations thereof. Additionally or alternatively, the antigens of this invention may be generated recombinantly.

PCR techniques may be exploited to selectively amplify the appropriate antigen (for example *S. uberis* antigen) gene sequences from a variety of sources including, for example, stored *Streptococcus* and/or *S. uberis* isolates, clinical isolates, diseased material and the like. Cloned antigen sequences may be introduced into a vector (such as a plasmid or expression cassette). In one embodiment, the vector may further comprise a nucleotide sequence of a tag or label to assist in protein purification procedures.

A host cell may be transformed or transfected with a vector and maintained under conditions suitable to induce expression of an antigen (for example a *S. uberis* antigen) gene sequence and production of recombinant antigen. Prokaryotic or eukaryotic cells, such as, for example bacterial, plant, insect, mammalian and/or fungal cells, may be transformed or transfected with one or more of the vectors described herein. One of skill in this field will be familiar with the techniques used to introduce heterologous or foreign nucleic acid sequences, such as expression vectors, into cells and these may include, for example, heat-shock treatment, use of one or more chemicals (such as calcium phosphate) to induce transformation/transfection, the use of viral carriers, microinjection and/or techniques such as electroporation. Further information regarding transformation/transfection techniques may be found in Current Protocols in Molecular Biology, Ausuble, F.M., ea., John Wiley & Sons, N.Y. (1989). In one embodiment, the host cell is a bacterial cell such as, for example, an *Escherichia coli* cell.

Techniques used to purify recombinant proteins generated in this way are known and, where the recombinant protein is tagged or labelled, these may include the use of, for example, affinity chromatography techniques.

In view of the above, there is described expression vectors comprising *S. uberis* antigen gene sequence(s) and host cells transformed therewith.

For convenience all of the antigens, including the *S. uberis* antigens (both purified and/or recombinant forms) described herein, shall hereinafter be collectively referred to as "antigens" or "*S. uberis* antigens". Moreover, references to specific antigens and/or *S. uberis* antigens should be taken to include (immunogenic) fragments or portions derived therefrom (as described above) as well as any mutants, variants and derivatives thereof and/or antigens exhibiting a degree of homology/identity thereto.

The inventors have discovered that animals (in particular bovine, porcine caprine and/or ovine animals) administered one or more of the *S. uberis* lipoprotein (acc No: YP_002562276), *S. uberis* serine proteinase (acc No: YP_002563137), *S. uberis* ferrichrome binding protein: (acc No: YP_002561776) and/or *S. uberis* elongation factor Tu: (acc No: YP_002561947) antigens, elicit particularly effective protective immune responses.

As such, there is described immunogenic and/or vaccine compositions comprising one or more of the *S. uberis* lipoprotein (acc No: YP_002562276), *S. uberis* serine proteinase (acc No: YP_002563137), *S. uberis* ferrichrome binding protein: (acc No: YP_002561776) and/or *S. uberis* elongation factor Tu: (acc No: YP_002561947).

Any of the antigens or *S. uberis* antigens described herein may be admixed with one or more other components, such as another polypeptide and/or an adjuvant or diluent. Additionally, or alternatively, compositions provided by this invention may, for example, contain viral, fungal, bacterial or other parasite antigens used to control other diseases/infections or infestations. For example, the composition may be included within a multivalent vaccine, which includes antigens against other ovine or bovine pathogens/diseases.

The term "adjuvant" generally refers to any material that increases the humoral or cellular immune response to an antigen. Adjuvants are used to accomplish two objectives: They slow the release of antigens from the injection site, and they enhance stimulation of the immune system. The addition of an adjuvant may permit the use of a smaller dose of antigen to stimulate a similar immune response, thereby reducing the production cost of the vaccine. Thus, the effectiveness of some injectable medicinal agents may be significantly increased when the agent is combined with an adjuvant.

In view of the above, the present invention may provide a composition comprising a *S. uberis* antigen having the amino acid sequence of SEQ ID NO:4 and a *S. uberis* antigen having the amino acid sequence of SEQ ID NO:2 and an adjuvant formulation.

As disclosed herein, the *S. uberis* antigen component of a composition may comprise, consist essentially of or consist of a *S. uberis* ferrichrome binding protein, *S. uberis* elongation factor Tu, *S. uberis* lipoprotein, *S. uberis* serine proteinase, an immunogenic fragment of any of these or a combination thereof.

Also described herein is that the compositions may comprise, consist essentially of or consist of *S. uberis* ferrichrome binding protein, *S. uberis* elongation factor Tu, *S. uberis* lipoprotein, *S. uberis* serine proteinase, an immunogenic fragment of any of these or a combination thereof optionally in combination with an adjuvant.

There is described a vaccine which may comprise, consist essentially of or consist of a *S. uberis* ferrichrome binding protein, *S. uberis* elongation factor Tu, *S. uberis* lipoprotein, *S. uberis* serine proteinase, an immunogenic fragment of any of these or a combination thereof optionally in combination with an adjuvant for use in treating masititis or raising an immune response which is protective against mastitis. The vaccine may be further exploited in methods of treating subjects in need thereof, the method comprising administering an immunologically effective amount of the vaccine to the subject. The subject may be a bovine subject or any subject (bovine or otherwise) predisposed or susceptible to contracting or developing mastitis. The subject may be suffering from mastitis. The subject may be predisposed or susceptible to contracting or developing a *S. uberis* infection or a *S. uberis* associated disease and/or condition.

The adjuvant (optionally to be used with any of the vaccine formulations disclosed herein) may be any suitable adjuvant. The adjuvant may not be an adjuvant formulation comprising, consisting essentially of or consisting of an oily phase and an aqueous phase, wherein the oily phase comprises at least 50% of the formulation v/v, wherein said formulation comprises at least one of monophosphoryl lipid A (MPL-A) or an analog thereof and an immunostimulatory oligonucleotide, with provisos that a) if said immunostimulatory oligonucleotide is absent, then the formulation comprises a poly I:C, a glycolipid, and, optionally, a quaternary amine; or a polycationic carrier; and b) if said monophosphoryl lipid A (MPL-A) or the analog thereof is absent, then the formulation comprises a source of aluminum, and, optionally, a polycationic carrier.

Polypeptides which may be used in conjunction with any or all of the antigens of this invention may include, for example polypetides which are fused, bound or conjugated to the *S. uberis* antigens described herein. Thus this invention further encompasses fusions comprising the *S. uberis* antigens described herein. The fusions may be internal fusions where a peptide or protein is embedded into the amino acid sequence of an antigen of this invention. Additionally or alternatively, the fusions may comprise C- or N-terminal fusions in which a peptide or protein is fused to the N- and/or C-terminal portion of a *S. uberis* antigen of this invention. In some cases, the *S. uberis* antigens (or the fragments thereof) of this invention may take the form of haptens - that is to say they are small molecules which elicit immune responses only when attached to a large carrier such as a peptide or protein. In some cases, the carier protein or peptide may not elicit an immune response by itself. Where the *S. uberis* antigen is a hapten, it may be fused, combined, bound or conjugated with or to a carrier protein or peptide so as to enhance or increase its ability to raise an immune response in an animal.

Described herein is an animal population treated, vaccinated and/or immunised with an antigen or antigen(s), vaccine or composition described herein. For example, there is described treated, vaccinated or immunised human, avian, piscine, bovine, porcine, caprine and/or ovine populations; for example, farmed populations of birds, fish, cattle, pigs sheep and/or goats may have been treated, vaccinated and/or immunised with an antigen or antigen(s), vaccine or composition described herein. As stated, a vaccine or composition may comprise one or more of the *S. uberis* antigens described herein optionally in combination with one or more other antigens and/or adjuvants.

The compositions, provided by this invention may be formulated as sterile pharmaceutical compositions comprising a pharmaceutical excipient, carrier or diluent. These composition may be formulated for oral, topical (including dermal and sublingual), intramammary, parenteral (including subcutaneous, intradermal, intramuscular and intravenous), transdermal and/or mucosal administration.

The compositions described herein, may comprise a discrete dosage unit and may be prepared by any of the methods well-known in the art of pharmacy. Methods typically include the step of bringing into association one or more of the *S. uberis* antigens described herein with liquid carriers or finely-divided solid carriers or both.

Compositions (the term "composition" including immunogenic and vaccine compositions of this invention), suitable for oral administration , wherein the carrier is a solid, are most preferably presented as unit dose formulations such as boluses, capsules or tablets each containing a predetermined amount of the *S. uberis* antigens. A tablet may be made by compression or moulding, optionally with one or more accessory ingredients. Compressed tablets may be prepared by compressing in a suitable machine an active compound (for example one or more *S. uberis* antigen(s)) in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersible granules, which may for example be suspended in water before administration, or sprinkled on food. The granules may be packaged, for example in a sachet. Formulations suitable for oral administration wherein the carrier is a liquid may be presented as a solution or a suspension in an aqueous or non-aqueous liquid, or as an oil-in-water liquid emulsion.

Compositions suitable for oral administration include controlled release dosage forms, for example tablets wherein an active compound (for example one or more *S. uberis* antigens) is formulated in an appropriate release-controlling matrix, or is coated with a suitable release-controlling film. Such compositions may be particularly convenient for prophylactic use.

Compositions formulated for parenteral administration include sterile solutions or suspensions of an active compound (for example one or more *S. uberis* antigens) in aqueous or oleaginous vehicles. Compositions of this invention, including vaccine and/or immunogenic compositions, may comprise, or further comprise cryoprotectant compounds or compositions, preservative(s), antibiotics, adjuvants and the like.

Injectable compositions and vaccines may be adapted for bolus injection or continuous infusion. Such preparations are conveniently presented in unit dose or multi-dose containers, which are sealed after introduction of the formulation until required for use. Alternatively, an active compound (for example one or more *S. uberis* antigens) may be in powder form that is constituted with a suitable vehicle, such as sterile, pyrogen-free water or phosphate buffered saline PBS before use.

Compositions may also be formulated as long-acting depot preparations, which may be administered by intramuscular injection or by implantation, e.g. subcutaneously or intramuscularly. Depot preparations may include, for example, suitable polymeric or hydrophobic materials, or ion-exchange resins. They may also include preparations or adjuvants known to enhance the affinity and/or longevity of an animal (for example bovine, ovine or caprine) immune response, such as single or double emulsions of oil in water. Such long-acting compositions are particularly convenient for prophylactic use.

Compositions suitable (or formulated) for mucosal administration include compositions comprising particles for aerosol dispersion, or dispensed in drinking water. When dispensed, such compositions should desirably have a particle diameter in the range 10 to 200 microns to enable retention in, for example, the nasal cavity; this may be achieved by, as appropriate, use of a powder of a suitable particle size or choice of an appropriate valve. Other suitable compositions include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

It should be understood that, in addition to the carrier ingredients mentioned above, the various compositions described herein may include an appropriate one or more additional (pharmaceutically acceptable) carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

Pharmaceutically acceptable carriers are well known to those skilled in the art and include, but are not limited to, 0.1 M and preferably 0.05 M phosphate buffer or 0.8% saline. Additionally, pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Compositions suitable for topical formulation may be provided, for example, as gels, creams or ointments.

Compositions for veterinary use may conveniently be in either powder or liquid concentrate form. In accordance with standard veterinary formulation practice, conventional water-soluble excipients, such as lactose or sucrose, may be incorporated in the powders to improve their physical properties. Thus, particularly suitable powders of this invention comprise 50 to 100% w/w and preferably 60 to 80% w/w of the active ingredient(s) (for example one or more *S. uberis* antigens) and 0 to 50% w/w and preferably 20 to 40% (w/w) of conventional veterinary excipients. These powders may either be added to, for example, animal feed - perhaps by way of an intermediate premix, or diluted in animal drinking water.

Liquid concentrates may optionally further include an acceptable water-miscible solvent for veterinary use, for example polyethylene glycol, propylene glycol, glycerol, glycerol formal or such a solvent mixed with up to 30% (v/v) of ethanol. The liquid concentrates may be administered to the drinking water of animals.

In addition to providing *S. uberis* antigens for use in raising immune responses in animals, there is also described polyclonal and/or monoclonal antibodies (or antigen binding fragments thereof) that bind (or have affinity or specificity for) any of the antigens or *S. uberis* antigens described. Production and isolation of polyclonal/monoclonal antibodies specific for protein/peptide sequences is routine in the art, and further information can be found in, for example "Basic methods in Antibody production and characterisation" Howard & Bethell, 2000, Taylor & Francis Ltd. Such antibodies may be used in diagnostic procedures, to, for example detect or diagnose *S. uberis* infection/infestations in animal (for example bovine, caprine or ovine) species, as well as for passive immunisation.

Also described is a vaccine for use in preventing or controlling *Streptococcus* infections and/or associated diseases and/or conditions. The vaccines may find particular application in preventing or controlling mastitis (namely mastitis with a Streptococcal aetiology). The vaccines may be used to prevent or control *S. uberis* infections and/or associated diseases. The vaccines may also be used to prevent or control streptococcal infections and/or associated diseases in human, avian, piscine, bovine, porcine, ovine and/or caprine hosts. The vaccine may be a polypeptide or polynucleotide vaccine - the polypeptides and/or polynucleotides providing, or encoding, one or more of the (*S. uberis*) antigens described herein.

There is further described a method for immunising an animal against an infection or disease caused or contributed to by a *Streptococcus* species, said method comprising the step of administering a vaccine of the invention to a subject in need thereof. Subjects in need thereof may be animals suffering (or suspected of suffering from) a streptococcal infection or disease and/or animals regarded as susceptible or predisposed to such diseases and/or infections. For example, there is described methods of immunising a human, an avian, a piscine, a caprine, a porcine, an ovine and/or bovine subject against *S. uberis* infection/infestation and associated diseases, said method comprising the step of administering a vaccine to the human, avian, piscine, caprine, porcine, ovine or bovine subject. A subject in need thereof may be any animal subject suffering from mastitis.

The antigens and/or vaccines described may be exploited in the immunisation of animals against mastitis, wherein the mastitis results from, is associated with or is caused or contributed to by, a *Streptococcus* species (for example *S. uberis* or *S. dysgalactiae*) or infection therewith.

The antigens described may find further application as the basis of diagnostic or identification/detection tests for various *Streptococcus* species/strains including any species or strain which expresses any of the antigens described herein. For example, the antigens described may be applied to the detection, identification and/or diagnosis of *S. uberis, S. agalactiae, S. dysgalactiae,* and *S. parauberis* infections, and/or diseases caused thereby or associated therewith. The antigens may also be used in methods for detecting the presence of streptococci in samples.

Thus, there is disclosed an (*in vitro*) method of detecting, identifying and/or diagnosing a streptococcus spp., *S. uberis, S. agalactiae, S. dysgalactiae and*/*or S. parauberis* infection or a disease caused thereby or associated therewith, the method comprising detecting a level of one or more of the antigens described herein in a sample and/or detecting a level of an antibody with specificity for one or more of the antigens described herein, in a sample.

A sample may comprise any biological fluid or tissue including, for example, whole blood; red blood cells, serum; plasma; saliva; sweat; semen; milk, (disease) tissue biopsy; tissue scraping; tissue/organ wash/lavage; and/or faecal preparations. The term "sample" may further extend to samples of animal feed/drink, bedding or field samples such as soil and grass.

Methods of detecting levels of antigen or antibody in a sample may comprise immunological methods. For example, methods of detecting levels of antigen or antibody may exploit enzyme-linked immunosorbant assay (ELISA) techniques. By way of example, one or more of the antigens described herein may be immobilised to a substrate and the immobilised antigen(s) used to probe a sample for the presence of antibodies reactive thereto. After a suitable period of incubation between the immobilised antigen and the sample, the presence or absence of antibodies may be detected by means of a secondary binding agent (for example antibody) optionally conjugated to a detectable moiety, with specificity for antibodies generated by the relevant species - for example bovine, caprine or ovine antibodies. The presence of antibodies may indicate the presence of a *Streptococcus* (for example *S. uberis*) and/or an infection therewith or a *Streptococcus* (for example an *S. uberis*) associated disease or condition.

Alternatively, binding agents or antibodies with specificity to one or more of the antigens described herein may be immobilised onto a substrate. The substrate may then be used to probe a sample for the presence of one or more antigens to which the binding agents or antibodies bind. After a suitable period of incubation between the sample and the immobilised antibody, the substrate may be contacted with a secondary binding agent or antibody with specificity for the relevant antigens. The secondary binding agent or antibody may be conjugated to a detectable moiety. Alternatively, the immobilised binding agent:antigen:binding agent complexes may be further probed with a tertiary binding agent or antibody capable of binding to the secondary binding agent. The tertiary binding agent may be conjugated to a detectable moiety.

Other immunological techniques such as immunohistochemical staining may exploit binding agents (for example antibodies/ conjugated antibodies) with specificity for one or more of the antigens described herein, to detect the presence or absence of *Streptococci* (for example *S. uberis*) antigens in a sample, for example a tissue sample.

Molecular methods may also be used to detect the presence of any of the antigens described herein (for example *S. uberis* antigens) in a sample. For example, primer sequences designed to amplify sequences encoding one or more of the (S. *uberis*) antigens described may be used to probe samples for the presence of the relevant (for example *S. uberis*) nucleic acid. Further information regarding these (PCR-based) techniques may be found in, for example, PCR Primer: A Laboratory Manual, Second Edition Edited by Carl W. Dieffenbach & Gabriela S. Dveksler: Cold Spring Harbour Laboratory Press and Molecular Cloning: A Laboratory Manual by Joseph Sambrook & David Russell: Cold Spring Harbour Laboratory Press.

Also disclosed are kits comprising reagents and compositions suitable for identifying or detecting the presence or absence of Streptococci (including *S. uberis, S. dysgalactiae and*/*or S. parauberis*) and/or diagnosing or detecting streptococci (including *S. uberis, S. dysgalactiae and*/*or S. parauberis*) infections or diseases. For example, depending on whether or not the kits are intended to be used to identify or detect streptococci and/or levels of antigen or antibodies thereto in samples, the kits may comprise substrates having antigens as disclosed herein or agents capable of binding the same, bound thereto. In addition, the kits may comprise agents capable of binding the relevant (for example *S. uberis)* antigens. Thus, where the kit is to be used to identify levels of *S. uberis* antigen in samples, the kit may comprise an agent capable of binding the relevant *S. uberis* antigen. The kit may comprise specifically raised polyclonal antibodies or monoclonal antibodies - the antibodies having specificity for antigens described herein. Where the kits are intended to diagnose or detect streptococci or streptococcal diseases in specific animals (for example human, avian, porcine, piscine, bovine, caprine and/or ovine animals) the kits may comprise binding agents or antibodies capable of binding immunoglobulin from the relevant species. The antibodies may be conjugated to detectable moieties. Kits for use in detecting the expression of genes encoding any of the antigens described herein may comprise one or more oligonucleotides/primers for detecting/amplifying/probing the relevant antigen-encoding sequences. The kits may also comprise other reagents to facilitate, for example, sequencing, PCR and/or RFLP analysis. All kits described herein may further comprise instructions for use.

### Example 1

### MATERIALS, METHODS & RESULTS

### Bacterial strains and culture conditions

The reference strain, *Streptococcus uberis* 0140J (ATCC BAA-854) was used in this study, in addition to a further 69 *S. uberis* clinical isolates derived from cases of bovine and ovine mastitis from distinct farms within the UK, Italy and the USA, and comprised strains which either persisted or were cured following antibiotic therapy. For routine culture, bacteria were propagated in Brain Heart Infusion (BHI) broth or agar. Bacteria for inclusion in proteomic analyses were propagated in a defined medium to provide a suitable growth environment lacking medium-derived peptides that would interfere with a mass spectrometric approach. Irrespective of the medium used, cultures were incubated static at 37°C.

### Analysis of S. uberis cell-wall and cell-wall-associated proteins

Twenty *S. uberis* strains, including the reference strain, were assessed in a proteomic analysis to identify putatively conserved proteins. Bacteria were propagated in 50 ml volumes to late exponential growth-phase; growth curves for each strain had been generated in a prior experiment, whereby growth was measured in the defined medium over time (data not shown). Bacterial cells were harvested by centrifugation at 30,000 × *g* for 20 m and washed twice with ice-cold PBS. Subsequently, in microcentrifuge tubes, the bacterial pellets were carefully re-suspended in 0.5 ml of PBS containing 40% (w/v) sucrose, 1mM DTT and 20µg sequencing grade modified trypsin (Promega). Proteolytic digestion of cells to liberate cell-wall and cell-wall-associated proteins was carried out for 2 h at 37°C with gentle shaking. Subsequently, the digestion mixtures were centrifuged at 30,000 × *g* for 10 m to pellet cells, and each supernatant was transferred to a fresh microcentrifuge tube. Incubation of supernatants was continued overnight at 37°C, then each was filtered through a 0.45 • m Spin-X centrifuge tube filter (Corning) and stored in a refrigerator until required.

### Mass spectrometric analysis

Peptide mixtures were cleaned using a C5-Reversed Phase HPLC column. Subsequently, filtered samples were subjected to liquid chromatography-electrospray ionisation-tandem mass spectrometry (LC-ESI-MS/MS) analysis. Liquid chromatography was performed using an Ultimate 3000 nano-HPLC system (Dionex) comprising a WPS-3000-well plate micro auto-sampler, a FLM-3000 flow manager and column compartment, a UVD-3000 UV detector, an LPG-3600 dual-gradient micro-pump and an SRD-3600 solvent rack controlled by Chromeleon chromatography software (Dionex: http://www1.dionex.com). A micro-pump flow rate of 246 µl min⁻¹ was used in combination with a cap-flow splitter cartridge, affording a ¹/₈₂ flow split and a final flow rate of 3 µl min⁻¹ through a 5 cm x 200 µm ID monolithic reversed-phase column (Dionex/LC Packings) maintained at 50°C. Samples of 1-4 µl were applied to the column by direct injection. Peptides were eluted by the application of a 15 min linear gradient from 8-45% solvent B (80% (w/v) acetonitrile, 0.1% (v/v) formic acid) and directed through a 3 nl UV detector flow cell. LC was interfaced directly with an Esquire HCTplus™ 3-D high capacity ion trap mass spectrometer (Bruker Daltonics) via a low-volume (50 µl min⁻¹ maximum) stainless steel nebuliser (Agilent) and ESI. Parameters for tandem MS analysis were set as previously described (Batycka et al., 2006).

Deconvoluted MS/MS data was submitted to an in-house server running MASCOT (MATRIX SCIENCE), and searched against the fully-annotated *S. uberis* 0140J genome sequence (NC 012004) using the MASCOT search algorithm. To this end, the fixed- and variable-modifications selected were carbamidomethyl (C) and oxidation (M) respectively, and mass tolerance values for MS and MS/MS were set at 1.5Da and 0.5Da respectively. Molecular weight search (MOWSE) scores attained for individual protein identifications were inspected manually and considered significant only if two or more peptides were matched for each protein and identified peptide contained an unbroken "*b*" or "*y*" ion series of a minimum of four amino acid residues. An in-house software programme was used to process raw MASCOT data and generate non-redundant lists of proteins identified in each of the cell-wall sub-cellular fractions of the 20 *S. uberis* strains. Proteins which were present in 50% or greater of the 20 strains (Table 4a) were considered putative candidate antigens for vaccine development and were subjected to further study.

### Assessing carriage of candidate antigen-encoding genes among a larger panel of strains

To further appraise the conservation of proteins identified by mass spectrometry, PCR was used to determine the presence/absence of protein-coding sequences within the genomes of the larger panel of *S. uberis* strains. Genomic DNA of 69 *S. uberis* strains (including those strains assessed by mass spectrometry) was extracted from overnight cultures using the DNeasy Blood & Tissue Extraction Kit (Qiagen) as per the manufacturer's instructions for 'hard to lyse' Gram-positive bacteria. Oligonucleotide primer pairs (Table 5) were designed to allow PCR amplification of each of the target genes. PCR was conducted using Taq PCR MasterMix Kit (Qiagen), as per the manufacturer's instructions. Following PCR, amplicons were visualised over UV light following electrophoresis through 1% (w/v) agarose gels containing GelRed. In all cases a PCR product was observed, indicative of the antigen encoding gene being present in each of the analysed strains. Subsequently, selected PCR products were analysed further by sequencing to confirm the identity of the amplified sequences (data not shown).

Based upon the results of proteomic and genomic screening, 4 conserved targets were identified (Tables 4a, 5 and 6) and chosen for further assessment as candidate vaccine antigens.

### Production of recombinant antigens

Each of the genes were amplified by PCR (as previously) from S. *uberis* 0140J genomic DNA using oligonucleotide primers designed to include appropriate restriction endonuclease recognition sites to facilitate in-frame cloning into the expression plasmid (Table 6). For genes predicted to contain a secretion signal peptide-encoding sequence (as determined using SignalP V.3.0; Bendtsen *et al.* 2004), each forward primer was designed to anneal, in-frame, immediately after the predicted signal peptide-encoding sequence. PCR amplicons were initially cloned using the TOPO TA Cloning Kit (Life Technologies Corp.). Subsequently, the *S. uberis* genes were cleaved from the TOPO vector using the primer-encoded restriction endonuclease sites; digests were electrophoresed through 1% (w/v) agarose gels, and the desired fragments were excised and purified using the QIAquick Gel extraction Kit (Qiagen). Finally, each *S. uberis* gene was cloned into pET-15b expression plasmid (Novagen), to allow expression of each protein with an N-terminal 6 x histidine (his) residue tag to facilitate downstream purification.

**Table 4a. List of proteins conserved amongst isolates selected by proteomic analysis.**

| **Locus tag** | **Protein** | **Conservation (%)** |
|---|---|---|
| **SUB0423** | ferrichrome binding protein | 80 |
| **SUB0604** | elongation factor Tu | 50 |
| **SUB0950** | Lipoprotein | 65 |
| **SUB1868** | serine protease | 100 |

**Table 5. Oligonucleotide primers used for screening S. uberis strains.**

| **Target gene** | **Sequence** (5 - to -3) | |
|---|---|---|
| **SUB0423** | Forward primer | GTTCTAGGAGATTAGAATTCA |
| | Reverse primer | TTTGGTTTGTGTCCGTCATAA |
| **SUB0604** | Forward primer | AGTAAGGTAAAGTTAGACTGTATTG |
| | Reverse primer | AGTTGTCTGACTCTAATTGTTAATC |
| **SUB0950** | Forward primer | GTTATTGGCCATAAGGCTA |
| | Reverse primer | TAAGGTCGCTCCACATTT |
| **SUB1868** | Forward primer | AGGTAATGCCGTGTCTA |
| | Reverse primer | ATGAATCCGAGGTTGGTA |

**Table 6. PCR amplification of candidate antigen-encoding genes.**

| **Target gene*** | **Primer name** | **Restriction site added** | **Primer sequence** (5 - to -3) |
|---|---|---|---|
| **SUB0423** | | | |
| ferrichrome binding protein | SUB0423_NSP_FX | XhoI | CGCGCGCTCGAGATGTCACAAAGCACAAAG |
| YP_002561776 | SUB0423_NSP_RB | BamHI | CGCGCGGGATCCCTAGTTGTGAGTTTTCTG |
| **SUB0604** | | | |
| elongation factor Tu | SUB0604_FX | XhoI | CGCGCGCTCGAGATGGCAAAAGAAAAATAC |
| YP_002561947 | SUB0604_R | BamHI | CGCGCGGGATCCTTAAGCTTCGATTTCTGA |
| **SUB0950** | | | |
| lipoprotein | SUB0950_NSP_FX | XhoI | CGCGCGCTCGAGATGGATAGCAAAGATGCT |
| YP_002562276 | SUB0950_NSP_RB | BamHI | CGCGCGGGATCCTTATTATTTTTCAGGAACTTT |
| **SUB1868** | | | |
| serine proteinase | SUB_NSP_FN | NdeI | CGCGCGCATATGACAAATCTTAATAAC |
| YP_002563137 | SUB_NSP_RX | XhoI | CGCGCGCTCGAGTTATTTTTCTAAATCTTTGGT |

| | | | |
|---|---|---|---|
| *Target genes are listed according to their locus tag within the *S. uberis* 0140J genome (accession number NC 012004), and the name and accession number of the products they encode. | | | |

Preliminary appraisal of the expression of each of the 14 proteins was conducted). Following electrophoresis through polyacrylamide gels, recombinant products were excised and subjected to MALDI-ToF MS. Mass spectrometric data was searched against the fully-annotated *S. uberis* 0140J genome sequence (NC 012004) using the MASCOT search algorithm to confirm the identities of the recombinant proteins (data not shown). Subsequently, 4 were chosen (based on the level of expression) for assessment in a preliminary vaccination experiment.

### Up-scaled expression of candidate antigens

The 4 antigens chosen for further study were (N.B. 'r' prefix denotes recombinant product) rSUB423 (ferrichrome binding protein), rSUB604 (elongation factor Tu), rSUB950 (lipoprotein) and rSUB1868 (serine protease). The coding sequences of each of the 4 antigens, and the corresponding translated amino acid sequences are presented in Table 3 (above).

Starter cultures of *Escherichia coli* BL21(DE3) containing each of the 4 recombinant plasmids were propagated in Lysogenic Broth (LB) containing 50 µg/ml of carbenicillin, overnight at 37°C with shaking. These were then used to inoculate 1 l volumes of LB (containing 50 µg/ml of carbenicillin) in 5 l conical flasks. Cultures were shaken at 37°C until an OD₆₀₀ₙₘ of 0.6 was reached. Expression of the recombinant genes was induced by supplementation of cultures with IPTG to a final concentration of 1 mM; incubation was then continued as before for a further 1 h, then rifampicin was added to a final concentration of 150 µg/ml and incubation was continued for a further 3 h. Cells were harvested by centrifugation at 12,000 × g for 15 m at 4°C, and cell pellets containing recombinant proteins were retained. Two of the recombinant proteins (rSUB950 and rSUB1868) remained soluble during expression and could be purified under native conditions. In contrast, the remaining proteins (rSUB423 and rSUB604) formed inclusion bodies during extraction and required purification under denaturing conditions.

### Protein purification under native conditions

The cell pellets containing rSUB950 and rSUB1868 were re-suspended in 20 ml each of lysis buffer (1× BugBuster protein extraction reagent (Merck Millipore), 50 mM Tris HCl pH 8.0, 500 mM NaCl, 10 mM imidazole, 25 U/ml Benzonase enzyme (Merck Millipore) and 1× Complete EDTA-free protease inhibitor cocktail (Roche Applied Science)) and incubated at 37°C for 60 min to allow cell lysis and degradation of nucleic acids. Cell lysates were centrifuged at 22,000 × *g* for 30 m at 4°C to pellet cell debris. Then, recombinant proteins in the clarified supernatants were recovered by immobilized metal ion affinity chromatography (IMAC) using Ni-CAM resin (Sigma). For each protein, 2 × 12 ml Eco-Pac Chromatography Columns (Bio-Rad) were loaded with 2 ml (bed volume) resin and washed by gravity flow with 10 ml of Equilibration Buffer (50 mM Tris HCl pH 8.0, 500 mM NaCl, 10 mM imidazole, 1× Complete EDTA-free protease inhibitor cocktail). The outlet of each column was sealed, and for each clarified lysate, 10 ml was added to each of 2 columns prior to sealing the inlet of each column with Parafilm (VWR). Columns were incubated on a tube rotator, overnight at 4°C. After incubation, columns were drained by gravity flow, and washed with 8 × 5 ml of Wash Buffer (50 mM Tris HCl pH 8.0, 500 mM NaCl, 10 mM imidazole, 1× Complete EDTA-free protease inhibitor cocktail). The recombinant protein in each column, bound to the Ni-CAM resin via the N-terminal 6 × his tag, was eluted in 5 × 2 ml of Elution Buffer (50 mM Tris HCl pH 8.0, 500 mM NaCl, 250 mM imidazole, 1× Complete EDTA-free protease inhibitor cocktail). Subsequently, the eluate of equivalent proteins was pooled, and each protein was concentrated using Amicon Ultra-15, 10 kDa Mᵣ cut-off, centrifugal filter units, as per the manufacturer's instructions.

### Purification under denaturing conditions

Cell pellets containing inclusion bodies were initially treated according to the native extraction protocol. Subsequently, cell pellets containing inclusion bodies were suspended in 20 ml of native lysis buffer. Lysozyme was added to a final concentration of 1 kU/ml and digestion was carried out at room temperature for 15 min. After incubation, an equal volume of BugBuster reagent (diluted in distilled water) was added to the suspensions, which were mixed by vortexing for 1 min and centrifuged at 5,000 × *g* for 15 min at 4°C to collect inclusion bodies. Inclusion body pellets were then washed a further 3 times with 1:10 diluted BugBuster, as previously. Finally, each inclusion body was dissolved in 20 ml of 8 M urea (pH 8.0) at room temperature for 15 m, and then centrifuged at 5,000 × *g* for 15 m at room temperature to pellet (remove) insoluble material. Subsequently, purification of each protein was performed using Ni-CAM resin, as described elsewhere except that the column buffers used for native purification were replaced with: Equilibration Buffer (0.1 M sodium phosphate, 8 M urea pH 8.0), Wash Buffer (0.1 M sodium phosphate, 8 M urea pH 6.3), and Elution Buffer (0.1 M sodium phosphate, 8 M urea pH 4.5).

### Size exclusion high-performance liquid chromatography (HPLC)

Size exclusion HPLC was performed using a Superose 12 10/300GL column (GE Healthcare) pre-equilibrated with 1× PBS, pH6.8 (for native, soluble proteins) or 8 M urea in 1 × PBS, pH6.8 (for denatured, insoluble proteins). Individual injections of 200 ml were applied to the column and proteins were resolved over a period of 60 m at a flow rate of 0.5 ml/m. The proteins that eluted from the column were monitored spectrophotometrically at a wavelength of 280nm. For each protein, fractions of 1 ml were collected, and those corresponding to peaks of UV-absorbent material were examined for the presence of protein by SDS-PAGE and Coomassie Brilliant Blue staining. For each protein, eluted fractions observed to contain an enriched source of the protein of interest were pooled and concentrated by centrifugation with amicon ultra filters (10kDa cut off).

Each of the 4 recombinant proteins was subjected to MALD-TOF MS, and mass spectrometric data was searched against the fully-annotated *S. uberis* 0140J genome sequence (NC 012004) using the MASCOT search algorithm to confirm that the identities of the recombinant proteins were as expected (data not shown).

### SUMMARY

In order to facilitate the development of a new mastitis vaccine, we have conducted a study to identify those proteins which are produced by a diverse sub-set of the *S. uberis* population. The work was conducted with no prior assumption that any particular class of protein (*e.g.* putative virulence factor) would signify a better vaccine candidate than any other class of protein, but rather that conservation between species was of primary importance.

Preliminary proteomic analysis of the cell-wall sub-cellular fraction of a number of diverse *S. uberis* strains allowed the identification of a panel of candidate antigens. Subsequently, the carriage of the genes encoding these antigens was assessed among a wider population of *S. uberis* strains. In so doing, the panel of candidate antigens was refined further, and proof of concept that these antigens could be used as vaccine(s) was obtained by production of recombinant derivatives of 4 of the proteins, and using these to successfully protect dairy cattle against mastitis following experimental heterologous challenge with *S. uberis (see* **Example 2** below).

### Example 2

### S. uberis vaccines in cattle

*Streptococcus uberis* is Gram-positive bacterium, with a cell wall structure similar to *Staphylococcus* spp., as well as other streptococci such as *S. agalactiae* and *S. dysgalactiae. Streptococcus uberis* is the most common *Streptococcus* species isolated from cases of mastitis. The *S. uberis* is found in the udder, in the intestine, and on the cow's skin and teats, which is where most streptococci tend to be. The particularity of *S. uberis* is its extraordinary ability to contaminate the external environment, *i.e.* in the bedding or anywhere on an animal. The contamination can take place during milking or from subsequent contact with *S. uberis* elsewhere in the environment. The particular ecology of *S. uberis* makes it particularly difficult to fight against this bacterium.

### Materials, methods and results

### Antigens

The potential antigens, described above, were used for vaccination of cows against *S. uberis.* Specifically, the following antigens were selected for the study:
SUB0423-ferrichrome binding protein
SUB0604 - elongation factor Tu
SUB0950 - lipoprotein
SUB1868 - Serine protease

The treatments were as follows:

**Table 13**

| Group | Antigen | Concentration | Adjuvant |
|---|---|---|---|
| T01 | Saline | N/A | Saline : 0.85% NaCl |
| T02 | SUB0423 + SUB0604 | 75 ug/2 ml | TXO: CpG 250 ug (SEQ ID NO: 8)/Dextran DEAE 100 mg/Oil 51% v/v |
| T03 | SUB0950+SUB1868 | | |
| T04 | 0423+0604+0950+1868 | | |

Animals were allotted at day -7 and vaccinated on days zero and 28. Calving occurred on ∼day 49. Samples of blood and milk were taken on days zero, 7, 28, 35, 49, 63, 70, and 84. The cows were challenged on day 70.

All calves were born alive in groups T2 and T4. One calf (out of 10) in T01 was still born. One calf (out of 10) in T03 died due to dystocia.

The following milk quality scoring system was implemented to indicate severity of abnormal signs:
0 = Normal
1 = Flakes
2 = Slugs / Clots
3 = Stringy / Watery / Bloody

The results of milk evaluation are summarized in Table 17 below:

**Table 14**

| | Milk Appearance: At least 1 quarter with score equal or greater than 2? | | | | Total |
|---|---|---|---|---|---|
| | No | | Yes | | |
| | Number | % | Number | % | Number |
| T01 | 1 | 14.3 | 6 | 85.7 | 7 |
| T02 | 2 | 25 | 6 | 75 | 8 |
| T03 | 1 | 11.1 | 8 | 88.9 | 9 |
| T04 | 4 | 50 | 4 | 50 | 8 |

The following udder evaluation scoring system was implemented:
0 = Normal
1 = Slight swelling
2 = Moderate swelling
3 = Severe

The results of udder evaluation are summarized in Table 18 below:

**Table 15**

| | Udder Evaluation: At least 1 quarter with score equal or greater than 2? | | | | Total |
|---|---|---|---|---|---|
| | No | | Yes | | |
| | Number | % | Number | % | Number |
| T01 | 1 | 14.3 | 6 | 85.7 | 7 |
| T02 | 2 | 25 | 6 | 75 | 8 |
| T03 | 1 | 11.1 | 8 | 88.9 | 9 |
| T04 | 4 | 50 | 4 | 50 | 8 |

These results demonstrate that a combination antigen (as in T04) provides the best protection against mastitis, without affecting the calving of treated cows.

### References

1) HARDIE, J.M. 1986 Other streptococci. In Bergey's Manual of Systematic Bacteriology Vol. 2, ed. Sneath, P.H.A., Mair, N.S., Sharp, M.E. & Holt, J.G. pp. 1068-1071. Baltimore: Williams & Wilkins.
2) T.J. Coffey, G.D. Pullinger, R. Urwin, K.A. Jolley, S.M. Wilson, M.C. Maiden, J.A. Leigh First insights into the evolution of Streptococcus uberis: A multilocus sequence typing scheme that enables investigation of its population biology. Appl. Environ. Microbiol., 72 (2006), pp. 1420-1428
3). Williams AM, Collins MD. 1990. Molecular taxonomic studies on Streptococcus uberis types I and II. Description of Streptococcus parauberis sp. nov. J. Appl. Bacteriol. 68:485-490.
4). Nho SW, Hikima J, Cha IS, Park SB, Jang HB, del Castillo CS, Kondo H, Hirono I, Aoki T, Jung TS. 2011. Complete genome sequence and immunoproteomic analyses of the bacterial fish pathogen Streptococcus parauberis. Journal of Bacteriology. 193:3356-3366.
5) Altschul, S.F., Gish, W., Miller, W., Myers, E.W. & Lipman, D.J. (1990) "Basic local alignment search tool." J. Mol. Biol. 215:403-410

## Claims

1. A composition comprising an excipient and a *Streptococcus uberis* antigen having the amino acid sequence of SEQ ID NO: 4 and a *Streptococcus uberis* antigen having the amino acid sequence of SEQ ID NO: 2.

2. The composition of claim 1, wherein the composition further comprises one or more *Streptococcus uberis* antigens selected from the group consisting of:
(i) an antigen having the amino acid sequence of SEQ ID NO: 6 or an immunogenic fragment thereof
(ii) an antigen having an amino acid sequence at least 60%, 70%, 80% or 90% identical to the sequence of SEQ ID NO: 6;
(iii) an antigen having the amino acid sequence of SEQ ID NO: 8 or an immunogenic fragment thereof;
(iv) an antigen having the amino acid sequence at least 60%, 70%, 80% or 90% identical to the sequence of SEQ ID NO: 8.

3. The composition of any preceding claim, wherein the composition further comprises an adjuvant.

## Patentansprüche

1. Zusammensetzung, umfassend einen Excipienten und ein *Streptococcus uberis-*Antigen, das die Aminosäuresequenz von SEQ ID NO: 4 aufweist, und ein *Streptococcus uberis-Antigen,* das die Aminosäuresequenz von SEQ ID NO: 2 aufweist.

2. Zusammensetzung nach Anspruch 1, wobei die Zusammensetzung ferner ein oder mehrere *Streptococcus uberis-Antigene* aufweist, die ausgewählt sind aus der Gruppe bestehend aus:
(i) ein Antigen, das die Aminosäuresequenz von SEQ ID NO: 6 aufweist oder ein immunogenes Fragment davon
(ii) ein Antigen, das eine Aminosäuresequenz aufweist, die zu mindestens 60%, 70%, 80% oder 90% zu der SEQ ID NO: 6 identisch ist;
(iii) ein Antigen, das die Aminosäuresequenz von SEQ ID NO: 8 aufweist oder ein immunogenes Fragment davon;
(iv) ein Antigen, das die Aminosäuresequenz aufweist, die zu mindestens 60%, 70%, 80% oder 90% zu der SEQ ID NO: 8 identisch ist.

3. Zusammensetzung nach einem der vorhergehenden Ansprüche, wobei die Zusammensetzung ferner ein Adjuvans aufweist.

## Revendications

1. Composition comprenant un excipient et un antigène de *Streptococcus uberis* possédant la séquence d'acides aminés de la SEQ ID NO: 4 et un antigène de *Streptococcus uberis* possédant la séquence d'acides aminés de la SEQ ID NO: 2.

2. Composition selon la revendication 1, dans laquelle la composition comprend en outre un ou plusieurs antigènes de *Streptococcus uberis* choisis dans le groupe constitué de:
(i) un antigène possédant la séquence d'acides aminés de la SEQ ID NO: 6 ou un fragment immunogène de celui-ci;
(ii) un antigène possédant une séquence d'acides aminés au moins 60%, 70%, 80% ou 90% identique à la SEQ ID NO: 6;
(iii) un antigène possédant la séquence d'acides aminés de la SEQ ID NO: 8 ou un fragment immunogène de celui-ci;
(iv) un antigène possédant une séquence d'acides aminés au moins 60%, 70%, 80% ou 90% identique à la SEQ ID NO: 8.

3. Composition selon l'une quelconque des revendications précédentes, dans laquelle la composition comprend en outre un adjuvant.
